(19) Europäisches Patentamt
European Patent Office
Office européen des brevets

(11) **EP 3 851 121 A1**

(12) **EUROPEAN PATENT APPLICATION**
published in accordance with Art. 153(4) EPC

(43) Date of publication:
**21.07.2021 Bulletin 2021/29**

(51) Int Cl.:
*A61K 39/395* *(2006.01)*       *A61K 9/08* *(2006.01)*
*A61K 9/19* *(2006.01)*          *A61K 47/02* *(2006.01)*
*A61K 47/10* *(2017.01)*         *A61K 47/12* *(2006.01)*
*A61K 47/18* *(2017.01)*         *A61K 47/26* *(2006.01)*
*A61P 11/06* *(2006.01)*         *A61P 15/00* *(2006.01)*
*A61P 17/00* *(2006.01)*         *A61P 37/08* *(2006.01)*

(21) Application number: 19860824.2

(22) Date of filing: 13.09.2019

(86) International application number:
**PCT/JP2019/036239**

(87) International publication number:
**WO 2020/054871 (19.03.2020 Gazette 2020/12)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
**KH MA MD TN**

(30) Priority:  14.09.2018  JP 2018173103

(71) Applicant: **Mitsubishi Tanabe Pharma Corporation Osaka-shi,**
**Osaka 541-8505 (JP)**

(72) Inventors:
• **IKEMOTO, Keisuke**
  **Osaka-shi, Osaka 541-8505 (JP)**
• **MORI, Naoki**
  **Osaka-shi, Osaka 541-8505 (JP)**
• **SAITO, Hiroshi**
  **Osaka-shi, Osaka 541-8505 (JP)**
• **TANIMOTO, Masahiko**
  **Osaka-shi, Osaka 541-8505 (JP)**

(74) Representative: **Gill Jennings & Every LLP**
**The Broadgate Tower**
**20 Primrose Street**
**London EC2A 2ES (GB)**

(54) **HUMAN ANTI-IL-33 MONOCLONAL-ANTIBODY-CONTAINING PHARMACEUTICAL COMPOSITION**

(57)    The purpose of the present invention is to provide a pharmaceutical composition containing a human anti-IL-33 monoclonal antibody suitable for administration to a subject. The present invention provides a pharmaceutical composition containing a human anti-IL-33 monoclonal antibody as an active ingredient, wherein the pharmaceutical composition is substantially free of sodium chloride or contains less than 30 mM of sodium chloride. The present invention also provides a freeze-dried form of the pharmaceutical composition.

FIG. 2

Effect of salt against clouding of antibody solution

| | H₂O | A5N | A5S | H6N | H6S | P6N | P6S | P7N | P7S |
|---|---|---|---|---|---|---|---|---|---|
| Clouding | − | + | − | + | − | + | − | + | − |

+ : Clouding    − : No Clouding

EP 3 851 121 A1

[Sequence Listing]

SEQUENCE LISTING

<110> Mitsubishi Tanabe Pharma Corporation

<120> Pharmaceutical composition comprising human anti-IL-33 monoclonal
      antibody

<130> P190447

<150> JP 2018-173103
<151> 2018-09-14

<160> 47

<170> PatentIn version 3.5

<210> 1
<211> 446
<212> PRT
<213> Artificial Sequence

<220>
<223> A10-1C04 Heavy Chain

<400> 1

Glu Val Gln Leu Leu Glu Ser Gly Gly Gly Leu Val Gln Pro Gly Gly
1               5                   10                  15


Ser Leu Arg Leu Ser Cys Ala Ala Ser Gly Phe Thr Phe Ser Asp Tyr
            20                  25                  30


Tyr Met Asn Trp Val Arg Gln Ala Pro Gly Lys Gly Leu Glu Trp Val
            35                  40                  45


Ser Ser Ile Ser Arg Tyr Ser Ser Tyr Ile Tyr Tyr Ala Asp Ser Val
        50                  55                  60


Lys Gly Arg Phe Thr Ile Ser Arg Asp Asn Ser Lys Asn Thr Leu Tyr
65                  70                  75                  80


Leu Gln Met Asn Ser Leu Arg Ala Glu Asp Thr Ala Val Tyr Tyr Cys
                85                  90                  95


Ala Arg Asp Ile Gly Gly Met Asp Val Trp Gly Gln Gly Thr Leu Val
            100                 105                 110


Thr Val Ser Ser Ala Ser Thr Lys Gly Pro Ser Val Phe Pro Leu Ala
            115                 120                 125


Pro Ser Ser Lys Ser Thr Ser Gly Gly Thr Ala Ala Leu Gly Cys Leu
        130                 135                 140


Val Lys Asp Tyr Phe Pro Glu Pro Val Thr Val Ser Trp Asn Ser Gly

|   | 145 | | | 150 | | | 155 | | | 160 |
|---|---|---|---|---|---|---|---|---|---|---|

| Ala | Leu | Thr | Ser | Gly | Val | His | Thr | Phe | Pro | Ala | Val | Leu | Gln | Ser | Ser |
|-----|-----|-----|-----|-----|-----|-----|-----|-----|-----|-----|-----|-----|-----|-----|-----|
|     |     |     | 165 |     |     |     | 170 |     |     |     |     | 175 |     |     |     |

| Gly | Leu | Tyr | Ser | Leu | Ser | Ser | Val | Val | Thr | Val | Pro | Ser | Ser | Ser | Leu |
|-----|-----|-----|-----|-----|-----|-----|-----|-----|-----|-----|-----|-----|-----|-----|-----|
|     |     |     | 180 |     |     |     | 185 |     |     |     |     | 190 |     |     |     |

| Gly | Thr | Gln | Thr | Tyr | Ile | Cys | Asn | Val | Asn | His | Lys | Pro | Ser | Asn | Thr |
|     |     | 195 |     |     |     | 200 |     |     |     |     | 205 |     |     |     |     |

Lys Val Asp Lys Lys Val Glu Pro Lys Ser Cys Asp Lys Thr His Thr
 210             215             220

Cys Pro Pro Cys Pro Ala Pro Glu Leu Leu Gly Gly Pro Ser Val Phe
225             230             235             240

Leu Phe Pro Pro Lys Pro Lys Asp Thr Leu Met Ile Ser Arg Thr Pro
            245             250             255

Glu Val Thr Cys Val Val Val Asp Val Ser His Glu Asp Pro Glu Val
            260             265             270

Lys Phe Asn Trp Tyr Val Asp Gly Val Glu Val His Asn Ala Lys Thr
            275             280             285

Lys Pro Arg Glu Glu Gln Tyr Asn Ser Thr Tyr Arg Val Val Ser Val
            290             295             300

Leu Thr Val Leu His Gln Asp Trp Leu Asn Gly Lys Glu Tyr Lys Cys
305             310             315             320

Lys Val Ser Asn Lys Ala Leu Pro Ala Pro Ile Glu Lys Thr Ile Ser
            325             330             335

Lys Ala Lys Gly Gln Pro Arg Glu Pro Gln Val Tyr Thr Leu Pro Pro
            340             345             350

Ser Arg Asp Glu Leu Thr Lys Asn Gln Val Ser Leu Thr Cys Leu Val
            355             360             365

Lys Gly Phe Tyr Pro Ser Asp Ile Ala Val Glu Trp Glu Ser Asn Gly
            370             375             380

Gln Pro Glu Asn Asn Tyr Lys Thr Thr Pro Pro Val Leu Asp Ser Asp
385             390             395             400

Gly Ser Phe Phe Leu Tyr Ser Lys Leu Thr Val Asp Lys Ser Arg Trp
                405                 410                 415

Gln Gln Gly Asn Val Phe Ser Cys Ser Val Met His Glu Ala Leu His
            420                 425                 430

Asn His Tyr Thr Gln Lys Ser Leu Ser Leu Ser Pro Gly Lys
        435                 440                 445

<210>   2
<211>   215
<212>   PRT
<213>   Artificial Sequence

<220>
<223>   A10-1C04 Light Chain

<400>   2

Gln Ser Val Leu Thr Gln Pro Pro Ser Ala Ser Gly Thr Pro Gly Gln
1               5                   10                  15

Arg Val Thr Ile Ser Cys Thr Gly Ser Ser Ser Asn Ile Gly Ala Val
            20                  25                  30

Tyr Asp Val His Trp Tyr Gln Gln Leu Pro Gly Thr Ala Pro Lys Leu
        35                  40                  45

Leu Ile Tyr Arg Asn Asn Gln Arg Pro Ser Gly Val Pro Asp Arg Phe
        50                  55                  60

Ser Gly Ser Lys Ser Gly Thr Ser Ala Ser Leu Ala Ile Ser Gly Leu
65                  70                  75                  80

Arg Ser Glu Asp Glu Ala Asp Tyr Tyr Cys Gln Thr Tyr Asp Ser Ser
                85                  90                  95

Arg Trp Val Phe Gly Gly Gly Thr Lys Leu Thr Val Leu Gly Gln Pro
            100                 105                 110

Lys Ala Ala Pro Ser Val Thr Leu Phe Pro Pro Ser Ser Glu Glu Leu
        115                 120                 125

Gln Ala Asn Lys Ala Thr Leu Val Cys Leu Ile Ser Asp Phe Tyr Pro
        130                 135                 140

Gly Ala Val Thr Val Ala Trp Lys Ala Asp Ser Ser Pro Val Lys Ala
145                 150                 155                 160

Gly Val Glu Thr Thr Thr Pro Ser Lys Gln Ser Asn Asn Lys Tyr Ala

<pre>
                        165                    170                         175


        Ala Ser Ser Tyr Leu Ser Leu Thr Pro Glu Gln Trp Lys Ser His Arg
                    180                   185                   190


        Ser Tyr Ser Cys Gln Val Thr His Glu Gly Ser Thr Val Glu Lys Thr
                    195                   200                   205


        Val Ala Pro Thr Glu Cys Ser
            210                   215


        <210>  3
        <211>  449
        <212>  PRT
        <213>  Artificial Sequence

        <220>
        <223>  A23-1A05 Heavy Chain

        <400>  3

        Glu Val Gln Leu Leu Glu Ser Gly Gly Gly Leu Val Gln Pro Gly Gly
        1               5                   10                   15


        Ser Leu Arg Leu Ser Cys Ala Ala Ser Gly Phe Thr Phe Ser Asn Tyr
                    20                   25                   30


        Tyr Met His Trp Val Arg Gln Ala Pro Gly Lys Gly Leu Glu Trp Val
                35                   40                   45


        Ser Ser Ile Ser Ala Arg Ser Arg Tyr His Tyr Tyr Ala Asp Ser Val
                50                   55                   60


        Lys Gly Arg Phe Thr Ile Ser Arg Asp Asn Ser Lys Asn Thr Leu Tyr
        65                   70                   75                   80


        Leu Gln Met Asn Ser Leu Arg Ala Glu Asp Thr Ala Val Tyr Tyr Cys
                        85                   90                   95


        Ala Arg Leu Ala Thr Arg His Asn Ala Phe Asp Ile Trp Gly Gln Gly
                    100                   105                   110


        Thr Leu Val Thr Val Ser Ser Ala Ser Thr Lys Gly Pro Ser Val Phe
                    115                   120                   125


        Pro Leu Ala Pro Ser Ser Lys Ser Thr Ser Gly Gly Thr Ala Ala Leu
            130                   135                   140


        Gly Cys Leu Val Lys Asp Tyr Phe Pro Glu Pro Val Thr Val Ser Trp
        145                   150                   155                   160
</pre>

Asn Ser Gly Ala Leu Thr Ser Gly Val His Thr Phe Pro Ala Val Leu
                165             170             175

Gln Ser Ser Gly Leu Tyr Ser Leu Ser Ser Val Val Thr Val Pro Ser
            180             185             190

Ser Ser Leu Gly Thr Gln Thr Tyr Ile Cys Asn Val Asn His Lys Pro
            195             200             205

Ser Asn Thr Lys Val Asp Lys Lys Val Glu Pro Lys Ser Cys Asp Lys
            210             215             220

Thr His Thr Cys Pro Pro Cys Pro Ala Pro Glu Leu Leu Gly Gly Pro
225             230             235             240

Ser Val Phe Leu Phe Pro Pro Lys Pro Lys Asp Thr Leu Met Ile Ser
            245             250             255

Arg Thr Pro Glu Val Thr Cys Val Val Val Asp Val Ser His Glu Asp
            260             265             270

Pro Glu Val Lys Phe Asn Trp Tyr Val Asp Gly Val Glu Val His Asn
            275             280             285

Ala Lys Thr Lys Pro Arg Glu Glu Gln Tyr Asn Ser Thr Tyr Arg Val
290             295             300

Val Ser Val Leu Thr Val Leu His Gln Asp Trp Leu Asn Gly Lys Glu
305             310             315             320

Tyr Lys Cys Lys Val Ser Asn Lys Ala Leu Pro Ala Pro Ile Glu Lys
            325             330             335

Thr Ile Ser Lys Ala Lys Gly Gln Pro Arg Glu Pro Gln Val Tyr Thr
            340             345             350

Leu Pro Pro Ser Arg Asp Glu Leu Thr Lys Asn Gln Val Ser Leu Thr
            355             360             365

Cys Leu Val Lys Gly Phe Tyr Pro Ser Asp Ile Ala Val Glu Trp Glu
            370             375             380

Ser Asn Gly Gln Pro Glu Asn Asn Tyr Lys Thr Thr Pro Pro Val Leu
385             390             395             400

Asp Ser Asp Gly Ser Phe Phe Leu Tyr Ser Lys Leu Thr Val Asp Lys

405          410          415

Ser Arg Trp Gln Gln Gly Asn Val Phe Ser Cys Ser Val Met His Glu
        420           425          430

Ala Leu His Asn His Tyr Thr Gln Lys Ser Leu Ser Leu Ser Pro Gly
        435           440          445

Lys


<210> 4
<211> 216
<212> PRT
<213> Artificial Sequence

<220>
<223> A23-1A05 Light Chain

<400> 4

Gln Ser Val Leu Thr Gln Pro Pro Ser Ala Ser Gly Thr Pro Gly Gln
1           5           10          15

Arg Val Thr Ile Ser Cys Ser Gly Ser Ser Ser Asn Ile Gly Asn Asn
        20           25          30

Ala Val Ser Trp Tyr Gln Gln Leu Pro Gly Thr Ala Pro Lys Leu Leu
        35           40          45

Ile Tyr Ala Ser Asn Met Arg Val Ile Gly Val Pro Asp Arg Phe Ser
        50           55          60

Gly Ser Lys Ser Gly Thr Ser Ala Ser Leu Ala Ile Ser Gly Leu Arg
65           70          75           80

Ser Glu Asp Glu Ala Asp Tyr Tyr Cys Gly Ala Trp Asp Asp Ser Gln
          85          90          95

Lys Ala Leu Val Phe Gly Gly Gly Thr Lys Leu Thr Val Leu Gly Gln
          100         105        110

Pro Lys Ala Ala Pro Ser Val Thr Leu Phe Pro Pro Ser Ser Glu Glu
         115         120        125

Leu Gln Ala Asn Lys Ala Thr Leu Val Cys Leu Ile Ser Asp Phe Tyr
         130         135        140

Pro Gly Ala Val Thr Val Ala Trp Lys Ala Asp Ser Ser Pro Val Lys
         145         150        155        160

```
Ala Gly Val Glu Thr Thr Thr Pro Ser Lys Gln Ser Asn Asn Lys Tyr
                165             170                 175

Ala Ala Ser Ser Tyr Leu Ser Leu Thr Pro Glu Gln Trp Lys Ser His
                180             185                 190

Arg Ser Tyr Ser Cys Gln Val Thr His Glu Gly Ser Thr Val Glu Lys
                195             200                 205

Thr Val Ala Pro Thr Glu Cys Ser
        210             215
```

<210> 5
<211> 449
<212> PRT
<213> Artificial Sequence

<220>
<223> A25-2C02 Heavy Chain

<400> 5

```
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Asn Tyr
                20              25                  30

Tyr Met His Trp Val Arg Gln Ala Pro Gly Lys Gly Leu Glu Trp Val
            35              40                  45

Ser Ser Ile Ser Ala Arg Ser Ser Tyr Ile Tyr Tyr Ala Asp Ser Val
        50              55                  60

Lys Gly Arg Phe Thr Ile Ser Arg Asp Asn Ser Lys Asn Thr Leu Tyr
65              70                  75                  80

Leu Gln Met Asn Ser Leu Arg Ala Glu Asp Thr Ala Val Tyr Tyr Cys
                85                  90                  95

Ala Arg Leu Ala Thr Arg Asn Asn Ala Phe Asp Ile Trp Gly Gln Gly
            100                 105                 110

Thr Leu Val Thr Val Ser Ser Ala Ser Thr Lys Gly Pro Ser Val Phe
            115                 120                 125

Pro Leu Ala Pro Ser Ser Lys Ser Thr Ser Gly Gly Thr Ala Ala Leu
            130                 135                 140
```

32

```
Gly Cys Leu Val Lys Asp Tyr Phe Pro Glu Pro Val Thr Val Ser Trp
145             150             155             160

Asn Ser Gly Ala Leu Thr Ser Gly Val His Thr Phe Pro Ala Val Leu
            165             170             175

Gln Ser Ser Gly Leu Tyr Ser Leu Ser Ser Val Val Thr Val Pro Ser
            180             185             190

Ser Ser Leu Gly Thr Gln Thr Tyr Ile Cys Asn Val Asn His Lys Pro
        195             200             205

Ser Asn Thr Lys Val Asp Lys Lys Val Glu Pro Lys Ser Cys Asp Lys
    210             215             220

Thr His Thr Cys Pro Pro Cys Pro Ala Pro Glu Leu Leu Gly Gly Pro
225             230             235             240

Ser Val Phe Leu Phe Pro Pro Lys Pro Lys Asp Thr Leu Met Ile Ser
            245             250             255

Arg Thr Pro Glu Val Thr Cys Val Val Val Asp Val Ser His Glu Asp
            260             265             270

Pro Glu Val Lys Phe Asn Trp Tyr Val Asp Gly Val Glu Val His Asn
        275             280             285

Ala Lys Thr Lys Pro Arg Glu Glu Gln Tyr Asn Ser Thr Tyr Arg Val
    290             295             300

Val Ser Val Leu Thr Val Leu His Gln Asp Trp Leu Asn Gly Lys Glu
305             310             315             320

Tyr Lys Cys Lys Val Ser Asn Lys Ala Leu Pro Ala Pro Ile Glu Lys
            325             330             335

Thr Ile Ser Lys Ala Lys Gly Gln Pro Arg Glu Pro Gln Val Tyr Thr
        340             345             350

Leu Pro Pro Ser Arg Asp Glu Leu Thr Lys Asn Gln Val Ser Leu Thr
        355             360             365

Cys Leu Val Lys Gly Phe Tyr Pro Ser Asp Ile Ala Val Glu Trp Glu
    370             375             380

Ser Asn Gly Gln Pro Glu Asn Asn Tyr Lys Thr Thr Pro Pro Val Leu
385             390             395             400
```

Asp Ser Asp Gly Ser Phe Phe Leu Tyr Ser Lys Leu Thr Val Asp Lys
                405                 410                 415

Ser Arg Trp Gln Gln Gly Asn Val Phe Ser Cys Ser Val Met His Glu
                420                 425                 430

Ala Leu His Asn His Tyr Thr Gln Lys Ser Leu Ser Leu Ser Pro Gly
                435                 440                 445

Lys


<210> 6
<211> 216
<212> PRT
<213> Artificial Sequence

<220>
<223> A25-2C02 Light Chain

<400> 6

Gln Ser Val Leu Thr Gln Pro Pro Ser Ala Ser Gly Thr Pro Gly Gln
1               5               10                  15

Arg Val Thr Ile Ser Cys Ser Gly Ser Ser Ser Asn Ile Gly Arg Asn
            20                  25                  30

Ala Val Asn Trp Tyr Gln Gln Leu Pro Gly Thr Ala Pro Lys Leu Leu
            35                  40                  45

Ile Tyr Ala Ser Asn Met Arg Val Ser Gly Val Pro Asp Arg Phe Ser
        50                  55                  60

Gly Ser Lys Ser Gly Thr Ser Ala Ser Leu Ala Ile Ser Gly Leu Arg
65                  70                  75                  80

Ser Glu Asp Glu Ala Asp Tyr Tyr Cys Trp Ala Trp Asp Asp Ser Gln
                85                  90                  95

Lys Val Gly Val Phe Gly Gly Gly Thr Lys Leu Thr Val Leu Gly Gln
                100                 105                 110

Pro Lys Ala Ala Pro Ser Val Thr Leu Phe Pro Pro Ser Ser Glu Glu
        115                 120                 125

Leu Gln Ala Asn Lys Ala Thr Leu Val Cys Leu Ile Ser Asp Phe Tyr
        130                 135                 140

```
Pro Gly Ala Val Thr Val Ala Trp Lys Ala Asp Ser Ser Pro Val Lys
145             150             155             160

Ala Gly Val Glu Thr Thr Thr Pro Ser Lys Gln Ser Asn Asn Lys Tyr
                165             170             175

Ala Ala Ser Ser Tyr Leu Ser Leu Thr Pro Glu Gln Trp Lys Ser His
            180             185             190

Arg Ser Tyr Ser Cys Gln Val Thr His Glu Gly Ser Thr Val Glu Lys
            195             200             205

Thr Val Ala Pro Thr Glu Cys Ser
    210             215
```

<210> 7
<211> 449
<212> PRT
<213> Artificial Sequence

<220>
<223> A25-3H04 Heavy Chain

<400> 7

```
Glu Val Gln Leu Leu Glu Ser Gly Gly Gly Leu Val Gln Pro Gly Gly
1               5               10              15

Ser Leu Arg Leu Ser Cys Ala Ala Ser Gly Phe Thr Phe Ser Arg Tyr
            20              25              30

Tyr Met His Trp Val Arg Gln Ala Pro Gly Lys Gly Leu Glu Trp Val
            35              40              45

Ser Ser Ile Ser Ala Gln Ser Ser His Ile Tyr Tyr Ala Asp Ser Val
    50              55              60

Glu Gly Arg Phe Thr Ile Ser Arg Asp Asn Ser Lys Asn Thr Leu Tyr
65              70              75              80

Leu Gln Met Asn Ser Leu Arg Ala Glu Asp Thr Ala Val Tyr Tyr Cys
                85              90              95

Ala Arg Leu Ala Thr Arg Gln Asn Ala Phe Asp Ile Trp Gly Gln Gly
            100             105             110

Thr Leu Val Thr Val Ser Ser Ala Ser Thr Lys Gly Pro Ser Val Phe
            115             120             125
```

```
Pro Leu Ala Pro Ser Ser Lys Ser Thr Ser Gly Gly Thr Ala Ala Leu
    130                 135             140

Gly Cys Leu Val Lys Asp Tyr Phe Pro Glu Pro Val Thr Val Ser Trp
145                 150             155                 160

Asn Ser Gly Ala Leu Thr Ser Gly Val His Thr Phe Pro Ala Val Leu
                165             170                 175

Gln Ser Ser Gly Leu Tyr Ser Leu Ser Ser Val Val Thr Val Pro Ser
                180             185             190

Ser Ser Leu Gly Thr Gln Thr Tyr Ile Cys Asn Val Asn His Lys Pro
            195             200             205

Ser Asn Thr Lys Val Asp Lys Lys Val Glu Pro Lys Ser Cys Asp Lys
    210             215             220

Thr His Thr Cys Pro Pro Cys Pro Ala Pro Glu Leu Leu Gly Gly Pro
225             230             235                 240

Ser Val Phe Leu Phe Pro Pro Lys Pro Lys Asp Thr Leu Met Ile Ser
            245             250             255

Arg Thr Pro Glu Val Thr Cys Val Val Val Asp Val Ser His Glu Asp
            260             265             270

Pro Glu Val Lys Phe Asn Trp Tyr Val Asp Gly Val Glu Val His Asn
            275             280             285

Ala Lys Thr Lys Pro Arg Glu Glu Gln Tyr Asn Ser Thr Tyr Arg Val
    290             295             300

Val Ser Val Leu Thr Val Leu His Gln Asp Trp Leu Asn Gly Lys Glu
305             310             315                 320

Tyr Lys Cys Lys Val Ser Asn Lys Ala Leu Pro Ala Pro Ile Glu Lys
            325             330             335

Thr Ile Ser Lys Ala Lys Gly Gln Pro Arg Glu Pro Gln Val Tyr Thr
            340             345             350

Leu Pro Pro Ser Arg Asp Glu Leu Thr Lys Asn Gln Val Ser Leu Thr
            355             360             365

Cys Leu Val Lys Gly Phe Tyr Pro Ser Asp Ile Ala Val Glu Trp Glu
    370             375             380
```

```
Ser Asn Gly Gln Pro Glu Asn Asn Tyr Lys Thr Thr Pro Pro Val Leu
385             390             395             400

Asp Ser Asp Gly Ser Phe Phe Leu Tyr Ser Lys Leu Thr Val Asp Lys
            405             410             415

Ser Arg Trp Gln Gln Gly Asn Val Phe Ser Cys Ser Val Met His Glu
            420             425             430

Ala Leu His Asn His Tyr Thr Gln Lys Ser Leu Ser Leu Ser Pro Gly
        435             440             445

Lys


<210>  8
<211>  216
<212>  PRT
<213>  Artificial Sequence

<220>
<223>  A25-3H04 Light Chain

<400>  8

Gln Ser Val Leu Thr Gln Pro Pro Ser Ala Ser Gly Thr Pro Gly Gln
1               5               10              15

Arg Val Thr Ile Ser Cys Ser Gly Ser Ser Ser Asn Ile Gly Arg Asn
            20              25              30

Ala Val Asn Trp Tyr Gln Gln Leu Pro Gly Thr Ala Pro Lys Leu Leu
            35              40              45

Ile Tyr Ala Ser Asn Met Arg Arg Ser Gly Val Pro Asp Arg Phe Ser
        50              55              60

Gly Ser Lys Ser Gly Thr Ser Ala Ser Leu Ala Ile Ser Gly Leu Arg
65              70              75              80

Ser Glu Asp Glu Ala Asp Tyr Tyr Cys Ser Ala Trp Asp Asp Ser Gln
            85              90              95

Lys Val Val Val Phe Gly Gly Gly Thr Lys Leu Thr Val Leu Gly Gln
            100             105             110

Pro Lys Ala Ala Pro Ser Val Thr Leu Phe Pro Pro Ser Ser Glu Glu
        115             120             125
```

```
Leu Gln Ala Asn Lys Ala Thr Leu Val Cys Leu Ile Ser Asp Phe Tyr
    130                 135                 140

Pro Gly Ala Val Thr Val Ala Trp Lys Ala Asp Ser Ser Pro Val Lys
145                 150                 155                 160

Ala Gly Val Glu Thr Thr Thr Pro Ser Lys Gln Ser Asn Asn Lys Tyr
                165                 170                 175

Ala Ala Ser Ser Tyr Leu Ser Leu Thr Pro Glu Gln Trp Lys Ser His
            180                 185                 190

Arg Ser Tyr Ser Cys Gln Val Thr His Glu Gly Ser Thr Val Glu Lys
            195                 200                 205

Thr Val Ala Pro Thr Glu Cys Ser
    210                 215
```

<210> 9
<211> 449
<212> PRT
<213> Artificial Sequence

<220>
<223> A26-1F02 Heavy Chain

<400> 9

```
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Asn Tyr
            20                  25                  30

Tyr Met His Trp Val Arg Gln Ala Pro Gly Lys Gly Leu Glu Trp Val
        35                  40                  45

Ser Ser Ile Ser Ala Arg Ser Ser Tyr Leu Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Leu Ala Thr Arg His Val Ala Phe Asp Ile Trp Gly Gln Gly
            100                 105                 110

Thr Leu Val Thr Val Ser Ser Ala Ser Thr Lys Gly Pro Ser Val Phe
```

38

115                120             125

Pro Leu Ala Pro Ser Ser Lys Ser Thr Ser Gly Gly Thr Ala Ala Leu
    130             135             140

Gly Cys Leu Val Lys Asp Tyr Phe Pro Glu Pro Val Thr Val Ser Trp
145            150          155            160

Asn Ser Gly Ala Leu Thr Ser Gly Val His Thr Phe Pro Ala Val Leu
          165          170            175

Gln Ser Ser Gly Leu Tyr Ser Leu Ser Ser Val Val Thr Val Pro Ser
          180          185          190

Ser Ser Leu Gly Thr Gln Thr Tyr Ile Cys Asn Val Asn His Lys Pro
          195          200          205

Ser Asn Thr Lys Val Asp Lys Lys Val Glu Pro Lys Ser Cys Asp Lys
    210             215            220

Thr His Thr Cys Pro Pro Cys Pro Ala Pro Glu Leu Leu Gly Gly Pro
225            230          235            240

Ser Val Phe Leu Phe Pro Pro Lys Pro Lys Asp Thr Leu Met Ile Ser
          245          250          255

Arg Thr Pro Glu Val Thr Cys Val Val Val Asp Val Ser His Glu Asp
          260          265          270

Pro Glu Val Lys Phe Asn Trp Tyr Val Asp Gly Val Glu Val His Asn
          275          280          285

Ala Lys Thr Lys Pro Arg Glu Glu Gln Tyr Asn Ser Thr Tyr Arg Val
          290          295          300

Val Ser Val Leu Thr Val Leu His Gln Asp Trp Leu Asn Gly Lys Glu
305            310          315            320

Tyr Lys Cys Lys Val Ser Asn Lys Ala Leu Pro Ala Pro Ile Glu Lys
          325          330          335

Thr Ile Ser Lys Ala Lys Gly Gln Pro Arg Glu Pro Gln Val Tyr Thr
          340          345          350

Leu Pro Pro Ser Arg Asp Glu Leu Thr Lys Asn Gln Val Ser Leu Thr
          355          360          365

```
Cys Leu Val Lys Gly Phe Tyr Pro Ser Asp Ile Ala Val Glu Trp Glu
    370             375             380

Ser Asn Gly Gln Pro Glu Asn Asn Tyr Lys Thr Thr Pro Pro Val Leu
385             390             395                         400

Asp Ser Asp Gly Ser Phe Phe Leu Tyr Ser Lys Leu Thr Val Asp Lys
                405             410             415

Ser Arg Trp Gln Gln Gly Asn Val Phe Ser Cys Ser Val Met His Glu
            420             425             430

Ala Leu His Asn His Tyr Thr Gln Lys Ser Leu Ser Leu Ser Pro Gly
        435             440             445

Lys
```

```
<210>  10
<211>  216
<212>  PRT
<213>  Artificial Sequence

<220>
<223>  A26-1F02 Light Chain

<400>  10
```

```
Gln Ser Val Leu Thr Gln Pro Pro Ser Ala Ser Gly Thr Pro Gly Gln
1               5               10              15

Arg Val Thr Ile Ser Cys Ser Gly Ser Ser Ser Asn Ile Gly Asn Asn
            20              25              30

Ala Val Asn Trp Tyr Gln Gln Leu Pro Gly Thr Ala Pro Lys Leu Leu
        35              40              45

Ile Tyr Ala Ser Asn Met Arg Arg Pro Gly Val Pro Asp Arg Phe Ser
    50              55              60

Gly Ser Lys Ser Gly Thr Ser Ala Ser Leu Ala Ile Ser Gly Leu Arg
65              70              75              80

Ser Glu Asp Glu Ala Asp Tyr Tyr Cys Glu Ala Trp Asp Asp Ser Gln
            85              90              95

Lys Ala Val Val Phe Gly Gly Gly Thr Lys Leu Thr Val Leu Gly Gln
            100             105             110

Pro Lys Ala Ala Pro Ser Val Thr Leu Phe Pro Pro Ser Ser Glu Glu
```

                    115                    120                    125

Leu Gln Ala Asn Lys Ala Thr Leu Val Cys Leu Ile Ser Asp Phe Tyr
    130                    135                    140

Pro Gly Ala Val Thr Val Ala Trp Lys Ala Asp Ser Ser Pro Val Lys
145                    150                    155                    160

Ala Gly Val Glu Thr Thr Thr Pro Ser Lys Gln Ser Asn Asn Lys Tyr
                165                    170                    175

Ala Ala Ser Ser Tyr Leu Ser Leu Thr Pro Glu Gln Trp Lys Ser His
                180                    185                    190

Arg Ser Tyr Ser Cys Gln Val Thr His Glu Gly Ser Thr Val Glu Lys
            195                    200                    205

Thr Val Ala Pro Thr Glu Cys Ser
    210                    215

<210>   11
<211>   5
<212>   PRT
<213>   Artificial Sequence

<220>
<223>   A10-1C04 H1

<400>   11

Asp Tyr Tyr Met Asn
1                   5

<210>   12
<211>   17
<212>   PRT
<213>   Artificial Sequence

<220>
<223>   A10-1C04 H2

<400>   12

Ser Ile Ser Arg Tyr Ser Ser Tyr Ile Tyr Tyr Ala Asp Ser Val Lys
1                   5                   10                   15

Gly

<210>   13
<211>   7
<212>   PRT
<213>   Artificial Sequence

<220>
<223>   A10-1C04 H3

<400>   13

Asp Ile Gly Gly Met Asp Val
1               5


<210>   14
<211>   14
<212>   PRT
<213>   Artificial Sequence

<220>
<223>   A10-1C04 L1

<400>   14

Thr Gly Ser Ser Ser Asn Ile Gly Ala Val Tyr Asp Val His
1               5                   10


<210>   15
<211>   7
<212>   PRT
<213>   Artificial Sequence

<220>
<223>   A10-1C04 L2

<400>   15

Arg Asn Asn Gln Arg Pro Ser
1               5


<210>   16
<211>   9
<212>   PRT
<213>   Artificial Sequence

<220>
<223>   A10-1C04 L3

<400>   16

Gln Thr Tyr Asp Ser Ser Arg Trp Val
1               5


<210>   17
<211>   5
<212>   PRT
<213>   Artificial Sequence

<220>
<223>   A23-1A05 H1, A25-2C02 H1 or A26-1F02 H1

<400>   17

Asn Tyr Tyr Met His

1                 5

<210> 18
<211> 17
<212> PRT
<213> Artificial Sequence

<220>
<223> A23-1A05 H2

<400> 18

Ser Ile Ser Ala Arg Ser Arg Tyr His Tyr Tyr Ala Asp Ser Val Lys
1                 5                 10                 15

Gly

<210> 19
<211> 10
<212> PRT
<213> Artificial Sequence

<220>
<223> A23-1A05 H3

<400> 19

Leu Ala Thr Arg His Asn Ala Phe Asp Ile
1                 5                 10

<210> 20
<211> 13
<212> PRT
<213> Artificial Sequence

<220>
<223> A23-1A05 L1

<400> 20

Ser Gly Ser Ser Ser Asn Ile Gly Asn Asn Ala Val Ser
1                 5                 10

<210> 21
<211> 7
<212> PRT
<213> Artificial Sequence

<220>
<223> A23-1A05 L2

<400> 21

Ala Ser Asn Met Arg Val Ile
1                 5

```
<210>    22
<211>    11
<212>    PRT
<213>    Artificial Sequence

<220>
<223>    A23-1A05 L3

<400>    22

Gly Ala Trp Asp Asp Ser Gln Lys Ala Leu Val
1               5                   10


<210>    23
<211>    17
<212>    PRT
<213>    Artificial Sequence

<220>
<223>    A25-2C02 H2

<400>    23

Ser Ile Ser Ala Arg Ser Ser Tyr Ile Tyr Tyr Ala Asp Ser Val Lys
1               5                   10                  15


Gly


<210>    24
<211>    10
<212>    PRT
<213>    Artificial Sequence

<220>
<223>    A25-2C02 H3

<400>    24

Leu Ala Thr Arg Asn Asn Ala Phe Asp Ile
1               5                   10


<210>    25
<211>    13
<212>    PRT
<213>    Artificial Sequence

<220>
<223>    A25-2C02 L1 or A25-3H04 L1

<400>    25

Ser Gly Ser Ser Ser Asn Ile Gly Arg Asn Ala Val Asn
1               5                   10


<210>    26
<211>    7
<212>    PRT
```

<210> Artificial Sequence

<220>
<223> A25-2C02 L2

<400> 26

Ala Ser Asn Met Arg Val Ser
1               5


<210> 27
<211> 11
<212> PRT
<213> Artificial Sequence

<220>
<223> A25-2C02 L3

<400> 27

Trp Ala Trp Asp Asp Ser Gln Lys Val Gly Val
1               5               10


<210> 28
<211> 5
<212> PRT
<213> Artificial Sequence

<220>
<223> A25-3H04 H1

<400> 28

Arg Tyr Tyr Met His
1               5


<210> 29
<211> 17
<212> PRT
<213> Artificial Sequence

<220>
<223> A25-3H04 H2

<400> 29

Ser Ile Ser Ala Gln Ser Ser His Ile Tyr Tyr Ala Asp Ser Val Glu
1               5               10              15


Gly


<210> 30
<211> 10
<212> PRT
<213> Artificial Sequence

<220>

<223>    A25-3H04 H3

<400>    30

Leu Ala Thr Arg Gln Asn Ala Phe Asp Ile
1               5                   10


<210>    31
<211>    7
<212>    PRT
<213>    Artificial Sequence

<220>
<223>    A25-3H04 L2

<400>    31

Ala Ser Asn Met Arg Arg Ser
1               5


<210>    32
<211>    11
<212>    PRT
<213>    Artificial Sequence

<220>
<223>    A25-3H04 L3

<400>    32

Ser Ala Trp Asp Asp Ser Gln Lys Val Val Val
1               5                   10


<210>    33
<211>    17
<212>    PRT
<213>    Artificial Sequence

<220>
<223>    A26-1F02 H2

<400>    33

Ser Ile Ser Ala Arg Ser Ser Tyr Leu Tyr Tyr Ala Asp Ser Val Lys
1               5                   10                  15

Gly


<210>    34
<211>    10
<212>    PRT
<213>    Artificial Sequence

<220>
<223>    A26-1F02 H3

<400>    34

```
Leu Ala Thr Arg His Val Ala Phe Asp Ile
1               5                   10
```

```
<210>   35
<211>   13
<212>   PRT
<213>   Artificial Sequence

<220>
<223>   A26-1F02 L1

<400>   35
```

```
Ser Gly Ser Ser Ser Asn Ile Gly Asn Asn Ala Val Asn
1               5                   10
```

```
<210>   36
<211>   7
<212>   PRT
<213>   Artificial Sequence

<220>
<223>   A26-1F02 L2

<400>   36
```

```
Ala Ser Asn Met Arg Arg Pro
1               5
```

```
<210>   37
<211>   11
<212>   PRT
<213>   Artificial Sequence

<220>
<223>   A26-1F02 L3

<400>   37
```

```
Glu Ala Trp Asp Asp Ser Gln Lys Ala Val Val
1               5                   10
```

```
<210>   38
<211>   116
<212>   PRT
<213>   Artificial Sequence

<220>
<223>   A10-1C04 Heavy Chain Variable Region

<400>   38
```

```
Glu Val Gln Leu Leu Glu Ser Gly Gly Gly Leu Val Gln Pro Gly Gly
1               5                   10                  15
```

```
Ser Leu Arg Leu Ser Cys Ala Ala Ser Gly Phe Thr Phe Ser Asp Tyr
                20                  25                  30
```

```
Tyr Met Asn Trp Val Arg Gln Ala Pro Gly Lys Gly Leu Glu Trp Val
    35                  40                  45

Ser Ser Ile Ser Arg Tyr Ser Ser Tyr Ile Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Asp Ile Gly Gly Met Asp Val Trp Gly Gln Gly Thr Leu Val
            100                 105                 110

Thr Val Ser Ser
        115
```

```
<210>  39
<211>  110
<212>  PRT
<213>  Artificial Sequence

<220>
<223>  A10-1C04 Light Chain Variable Region

<400>  39
```

```
Gln Ser Val Leu Thr Gln Pro Pro Ser Ala Ser Gly Thr Pro Gly Gln
1               5                   10                  15

Arg Val Thr Ile Ser Cys Thr Gly Ser Ser Ser Asn Ile Gly Ala Val
            20                  25                  30

Tyr Asp Val His Trp Tyr Gln Gln Leu Pro Gly Thr Ala Pro Lys Leu
        35                  40                  45

Leu Ile Tyr Arg Asn Asn Gln Arg Pro Ser Gly Val Pro Asp Arg Phe
        50                  55                  60

Ser Gly Ser Lys Ser Gly Thr Ser Ala Ser Leu Ala Ile Ser Gly Leu
65                  70                  75                  80

Arg Ser Glu Asp Glu Ala Asp Tyr Tyr Cys Gln Thr Tyr Asp Ser Ser
                85                  90                  95

Arg Trp Val Phe Gly Gly Gly Thr Lys Leu Thr Val Leu Gly
            100                 105                 110
```

```
<210>    40
<211>    119
<212>    PRT
<213>    Artificial Sequence

<220>
<223>    A23-1A05 Heavy Chain Variable Region

<400>    40

Glu Val Gln Leu Leu Glu Ser Gly Gly Gly Leu Val Gln Pro Gly Gly
1               5                   10                  15


Ser Leu Arg Leu Ser Cys Ala Ala Ser Gly Phe Thr Phe Ser Asn Tyr
            20                  25                  30


Tyr Met His Trp Val Arg Gln Ala Pro Gly Lys Gly Leu Glu Trp Val
        35                  40                  45


Ser Ser Ile Ser Ala Arg Ser Arg Tyr His Tyr Tyr Ala Asp Ser Val
        50                  55                  60


Lys Gly Arg Phe Thr Ile Ser Arg Asp Asn Ser Lys Asn Thr Leu Tyr
65                  70                  75                  80


Leu Gln Met Asn Ser Leu Arg Ala Glu Asp Thr Ala Val Tyr Tyr Cys
                85                  90                  95


Ala Arg Leu Ala Thr Arg His Asn Ala Phe Asp Ile Trp Gly Gln Gly
            100                 105                 110


Thr Leu Val Thr Val Ser Ser
            115


<210>    41
<211>    111
<212>    PRT
<213>    Artificial Sequence

<220>
<223>    A23-1A05 Light Chain Variable Region

<400>    41

Gln Ser Val Leu Thr Gln Pro Pro Ser Ala Ser Gly Thr Pro Gly Gln
1               5                   10                  15


Arg Val Thr Ile Ser Cys Ser Gly Ser Ser Ser Asn Ile Gly Asn Asn
            20                  25                  30


Ala Val Ser Trp Tyr Gln Gln Leu Pro Gly Thr Ala Pro Lys Leu Leu
        35                  40                  45
```

```
Ile Tyr Ala Ser Asn Met Arg Val Ile Gly Val Pro Asp Arg Phe Ser
    50                  55                  60

Gly Ser Lys Ser Gly Thr Ser Ala Ser Leu Ala Ile Ser Gly Leu Arg
65                  70                  75                  80

Ser Glu Asp Glu Ala Asp Tyr Tyr Cys Gly Ala Trp Asp Asp Ser Gln
                85                  90                  95

Lys Ala Leu Val Phe Gly Gly Gly Thr Lys Leu Thr Val Leu Gly
            100                 105                 110
```

```
<210>   42
<211>   119
<212>   PRT
<213>   Artificial Sequence

<220>
<223>   A25-2C02 Heavy Chain Variable Region

<400>   42
```

```
Glu Val Gln Leu Leu Glu Ser Gly Gly Gly Leu Val Gln Pro Gly Gly
1               5               10                  15

Ser Leu Arg Leu Ser Cys Ala Ala Ser Gly Phe Thr Phe Ser Asn Tyr
            20                  25                  30

Tyr Met His Trp Val Arg Gln Ala Pro Gly Lys Gly Leu Glu Trp Val
        35                  40                  45

Ser Ser Ile Ser Ala Arg Ser Ser Tyr Ile Tyr Tyr Ala Asp Ser Val
    50                  55                  60

Lys Gly Arg Phe Thr Ile Ser Arg Asp Asn Ser Lys Asn Thr Leu Tyr
65                  70                  75                  80

Leu Gln Met Asn Ser Leu Arg Ala Glu Asp Thr Ala Val Tyr Tyr Cys
                85                  90                  95

Ala Arg Leu Ala Thr Arg Asn Asn Ala Phe Asp Ile Trp Gly Gln Gly
            100                 105                 110

Thr Leu Val Thr Val Ser Ser
            115
```

```
<210>   43
<211>   111
<212>   PRT
```

<213> Artificial Sequence

<220>
<223> A25-2C02 Light Chain Variable Region

<400> 43

```
Gln Ser Val Leu Thr Gln Pro Pro Ser Ala Ser Gly Thr Pro Gly Gln
1               5                   10                  15

Arg Val Thr Ile Ser Cys Ser Gly Ser Ser Ser Asn Ile Gly Arg Asn
            20                  25                  30

Ala Val Asn Trp Tyr Gln Gln Leu Pro Gly Thr Ala Pro Lys Leu Leu
            35                  40                  45

Ile Tyr Ala Ser Asn Met Arg Val Ser Gly Val Pro Asp Arg Phe Ser
        50                  55                  60

Gly Ser Lys Ser Gly Thr Ser Ala Ser Leu Ala Ile Ser Gly Leu Arg
65                  70                  75                  80

Ser Glu Asp Glu Ala Asp Tyr Tyr Cys Trp Ala Trp Asp Asp Ser Gln
                85                  90                  95

Lys Val Gly Val Phe Gly Gly Gly Thr Lys Leu Thr Val Leu Gly
                100                 105                 110
```

<210> 44
<211> 119
<212> PRT
<213> Artificial Sequence

<220>
<223> A25-3H04 Heavy Chain Variable Region

<400> 44

```
Glu Val Gln Leu Leu Glu Ser Gly Gly Gly Leu Val Gln Pro Gly Gly
1               5                   10                  15

Ser Leu Arg Leu Ser Cys Ala Ala Ser Gly Phe Thr Phe Ser Arg Tyr
            20                  25                  30

Tyr Met His Trp Val Arg Gln Ala Pro Gly Lys Gly Leu Glu Trp Val
            35                  40                  45

Ser Ser Ile Ser Ala Gln Ser Ser His Ile Tyr Tyr Ala Asp Ser Val
        50                  55                  60

Glu Gly Arg Phe Thr Ile Ser Arg Asp Asn Ser Lys Asn Thr Leu Tyr
65                  70                  75                  80
```

```
Leu Gln Met Asn Ser Leu Arg Ala Glu Asp Thr Ala Val Tyr Tyr Cys
                85                  90                  95


Ala Arg Leu Ala Thr Arg Gln Asn Ala Phe Asp Ile Trp Gly Gln Gly
            100                 105                 110


Thr Leu Val Thr Val Ser Ser
            115
```

```
<210>   45
<211>   111
<212>   PRT
<213>   Artificial Sequence

<220>
<223>   A25-3H04 Light Chain Variable Region

<400>   45
```

```
Gln Ser Val Leu Thr Gln Pro Pro Ser Ala Ser Gly Thr Pro Gly Gln
1                5                  10                  15


Arg Val Thr Ile Ser Cys Ser Gly Ser Ser Ser Asn Ile Gly Arg Asn
            20                  25                  30


Ala Val Asn Trp Tyr Gln Gln Leu Pro Gly Thr Ala Pro Lys Leu Leu
            35                  40                  45


Ile Tyr Ala Ser Asn Met Arg Arg Ser Gly Val Pro Asp Arg Phe Ser
        50                  55                  60


Gly Ser Lys Ser Gly Thr Ser Ala Ser Leu Ala Ile Ser Gly Leu Arg
65                  70                  75                  80


Ser Glu Asp Glu Ala Asp Tyr Tyr Cys Ser Ala Trp Asp Asp Ser Gln
                85                  90                  95


Lys Val Val Val Phe Gly Gly Gly Thr Lys Leu Thr Val Leu Gly
            100                 105                 110
```

```
<210>   46
<211>   119
<212>   PRT
<213>   Artificial Sequence

<220>
<223>   A26-1F02 Heavy Chain Variable Region

<400>   46
```

```
Glu Val Gln Leu Leu Glu Ser Gly Gly Gly Leu Val Gln Pro Gly Gly
```

```
        1               5                   10                  15

        Ser Leu Arg Leu Ser Cys Ala Ala Ser Gly Phe Thr Phe Ser Asn Tyr
                    20              25              30

        Tyr Met His Trp Val Arg Gln Ala Pro Gly Lys Gly Leu Glu Trp Val
                    35              40              45

        Ser Ser Ile Ser Ala Arg Ser Ser Tyr Leu Tyr Tyr Ala Asp Ser Val
            50              55              60

        Lys Gly Arg Phe Thr Ile Ser Arg Asp Asn Ser Lys Asn Thr Leu Tyr
        65              70              75              80

        Leu Gln Met Asn Ser Leu Arg Ala Glu Asp Thr Ala Val Tyr Tyr Cys
                    85              90              95

        Ala Arg Leu Ala Thr Arg His Val Ala Phe Asp Ile Trp Gly Gln Gly
                    100             105             110

        Thr Leu Val Thr Val Ser Ser
                    115


        <210>   47
        <211>   111
        <212>   PRT
        <213>   Artificial Sequence

        <220>
        <223>   A26-1F02 Light Chain Variable Region

        <400>   47

        Gln Ser Val Leu Thr Gln Pro Pro Ser Ala Ser Gly Thr Pro Gly Gln
        1               5                   10                  15

        Arg Val Thr Ile Ser Cys Ser Gly Ser Ser Ser Asn Ile Gly Asn Asn
                    20              25              30

        Ala Val Asn Trp Tyr Gln Gln Leu Pro Gly Thr Ala Pro Lys Leu Leu
                    35              40              45

        Ile Tyr Ala Ser Asn Met Arg Arg Pro Gly Val Pro Asp Arg Phe Ser
            50              55              60

        Gly Ser Lys Ser Gly Thr Ser Ala Ser Leu Ala Ile Ser Gly Leu Arg
        65              70              75              80

        Ser Glu Asp Glu Ala Asp Tyr Tyr Cys Glu Ala Trp Asp Asp Ser Gln
                    85              90              95
```

```
Lys Ala Val Val Phe Gly Gly Gly Thr Lys Leu Thr Val Leu Gly
            100                105                110
```

**Description**

FIELD

The present invention relates to a human anti-IL-33 monoclonal antibody (A10-1C04, A23-1A05, A25-2C02, A25-3H04 or A26-1F02)-containing pharmaceutical composition, and in particular it relates to a pharmaceutical composition containing an antibody that inhibits clouding and improves storage stability, including pH stability.

BACKGROUND

A variety of antibody-containing pharmaceutical compositions have been developed and implemented in recent years, but most of antibody-containing pharmaceutical compositions are used as pharmaceutical compositions for intravenous injection. Due to ever-increasing needs at medical fields, there is a greater demand for developing antibody-containing pharmaceutical compositions for subcutaneous injection that are suitable for self-injection.

When designing antibody-containing pharmaceutical compositions for subcutaneous injection, it is necessary for the antibody dose per injection to be high (about 100 to 200 mg), and due to general restrictions for injection volumes by subcutaneous injection, the concentration of antibody in the dosing solution must be high. When a high-concentration antibody-containing pharmaceutical composition is prepared by redissolving a lyophilized pharmaceutical composition using water in a smaller amount than before lyophilization, it is most common for the high-concentration antibody-containing pharmaceutical composition used to be one obtained by lyophilized concentration technology.

The antibody subtype IgG which is commonly used in medicines is a high-molecular-weight glycoprotein of about 150 kDa having very high variety in its antibody variable region structure (amino acid sequence or sugar chain structure), and its properties therefore differ depending on the molecular species of the antibody.

Antibodies often become denatured by chemical reaction that occurs in response to heat or physical stimulation such as vibration, interaction with compounds such as surfactants, oxidation-reduction agents or sugars, or coagulation or decomposition due to prolonged storage. Antibody denaturation alters affinity for antigen or Fc receptors, thus attenuating the function and effect of the antibody and potentially resulting in inflammation being elicited by aggregated antibodies. The tendency toward aggregation or other forms of denaturation differs depending on the molecular species of the antibody.

Because antibodies include charged amino acids or highly polar amino acids and sugars, they interact with ions in antibody solutions, thus often forming a disproportional distribution of ions and altering the pH of the antibody solution (pH drift) (Donnan effect). Altered pH of an antibody solution sometimes has an effect on the storage stability of the antibody, and the strength of the Donnan effect differs depending on the molecular species of the antibody.

A high-concentration antibody solution tends to form a highly viscous solution due to the macromolecular properties of and molecular interactions between proteins. When the viscosity of an antibody solution increases it then becomes difficult to administer the antibody. The viscosity increase of an antibody solution due to high concentration of the antibody differs depending on the molecular species of the antibody.

When an antibody solution is stored for a prolonged period, changes in pH or degradation including formation of insoluble and/or soluble aggregates is an issue that must be dealt with, and this also differs depending on the molecular species of the antibody.

Antibody-containing pharmaceutical compositions are usually prepared with various modifications to obtain stable pharmaceutical compositions that have low loss of active ingredients even after prolonged storage, the active antibodies being dissolved together with various additives such as buffering agents to produce the pharmaceutical compositions. However, technology is not yet sufficient for preventing antibody aggregation, clouding, viscosity increase or pH drift in pharmaceutical compositions containing particularly high-concentrations of antibodies.

The present inventors have previously acquired human anti-IL-33 monoclonal antibodies that bind to IL-33 (PTL 1: International Patent Publication No. 2015/099175), but there is a need for development of pharmaceutical compositions that contain and are suitable for administration of these antibodies.

[CITATION LIST]

[PATENT LITERATURE]

[PTL 1] International Patent Publication No. 2015/099175

SUMMARY

[TECHNICAL PROBLEM]

It is an object of the invention to provide a pharmaceutical composition that contains a human anti-IL-33 monoclonal antibody suited for administration to a subject.

[SOLUTION TO PROBLEM]

The present inventors have found that human anti-IL-33 monoclonal antibody clouds when formulated, and that the clouding occurs due to formation of aggregates. The clouding was found to be dependent on NaCl concentration and pH. The present invention has been completed on the basis of this finding.

Specifically, the invention provides the following.

[1] A pharmaceutical composition comprising human anti-IL-33 monoclonal antibody as an active ingredient, wherein:

the combination of amino acid sequences in the heavy chain complementarity determining region 1 (H1), the heavy chain complementarity determining region 2 (H2), the heavy chain complementarity determining region 3 (H3), the light chain complementarity determining region 1 (L1), the light chain complementarity determining region 2 (L2) and the light chain complementarity determining region 3 (L3) of the human anti-IL-33 monoclonal antibody is one from among C1 to C5 listed in Table 1, and

the pharmaceutical composition contains substantially no sodium chloride or contains sodium chloride at less than 30 mM.

[Table 1]

Table 1 The following sequence ID Nos. are those of the Sequence Listing

|    | H1 | H2 | H3 | L1 | L2 | L3 |
|----|----|----|----|----|----|----|
| C1 | SEQ ID NO: 11 | SEQ ID NO: 12 | SEQ ID NO: 13 | SEQ ID NO: 14 | SEQ ID NO: 15 | SEQ ID NO: 16 |
| C2 | SEQ ID NO: 17 | SEQ ID NO: 18 | SEQ ID NO: 19 | SEQ ID NO: 20 | SEQ ID NO: 21 | SEQ ID NO: 22 |
| C3 | SEQ ID NO: 17 | SEQ ID NO: 23 | SEQ ID NO: 24 | SEQ ID NO: 25 | SEQ ID NO: 26 | SEQ ID NO: 27 |
| C4 | SEQ ID NO: 28 | SEQ ID NO: 29 | SEQ ID NO: 30 | SEQ ID NO: 25 | SEQ ID NO: 31 | SEQ ID NO: 32 |
| C5 | SEQ ID NO: 17 | SEQ ID NO: 33 | SEQ ID NO: 34 | SEQ ID NO: 35 | SEQ ID NO: 36 | SEQ ID NO: 37 |

[2] The pharmaceutical composition according to [1], wherein the combination of amino acid sequences of the human anti-IL-33 monoclonal antibody heavy chain variable region and light chain variable region is any one from among V1 to V5 listed in Table 2.

[Table 2]

Table 2 The following sequence ID Nos. are those of the Sequence Listing

|    | Heavy chain variable region | Light chain variable region |
|----|-----------------------------|-----------------------------|
| V1 | SEQ ID NO: 38 | SEQ ID NO: 39 |
| V2 | SEQ ID NO: 40 | SEQ ID NO: 41 |
| V3 | SEQ ID NO: 42 | SEQ ID NO: 43 |
| V4 | SEQ ID NO: 44 | SEQ ID NO: 45 |
| V5 | SEQ ID NO: 46 | SEQ ID NO: 47 |

[3] The pharmaceutical composition according to [1] or [2], wherein the human anti-IL-33 monoclonal antibody is A10-1C04, A23-1A05, A25-2C02, A25-3H04 or A26-1F02.

[4] The pharmaceutical composition according to any one of [1] to [3], wherein the sodium chloride concentration is 10 mM or lower.

[5] The pharmaceutical composition according to any one of [1] to [4], which contains substantially no sodium chloride.

[6] The pharmaceutical composition according to any one of [1] to [5], wherein the pH is adjusted to be higher than 4 and lower than 8.

[7] The pharmaceutical composition according to any one of [1] to [6], wherein the pH is adjusted to be 5 to 7.

[8] The pharmaceutical composition according to any one of [1] to [7], wherein the pH is adjusted by an acetate, histidine or phosphate buffer.

[9] The pharmaceutical composition according to any one of [1] to [8], wherein the pH is adjusted by histidine.

[10] The pharmaceutical composition according to any one of [1] to [9], wherein the concentration of the active ingredient is less than 175 mg/ml.

[11] The pharmaceutical composition according to any one of [1] to [10], wherein the concentration of the active ingredient is 150 mg/ml or lower.

[12] The pharmaceutical composition according to any one of [1] to [11], which contains at least one polyol.

[12-1] The pharmaceutical composition according to [12], wherein the polyol is a saccharide selected from the group consisting of disaccharides and sugar alcohols.

[13] The pharmaceutical composition according to [12] or [12-1], wherein the polyol is 3 to 5% (w/v) sorbitol.

[14] The pharmaceutical composition according to any one of [1] to [13], which contains a surfactant.

[15] The pharmaceutical composition according to [14], wherein the surfactant is a nonionic surfactant.

[15-1] The pharmaceutical composition according to [15], wherein the surfactant is polysorbate 20, polysorbate 80 or poloxamer 188.

[16] The pharmaceutical composition according to any one of [1] to [15-1], which includes 10 mM histidine, 4% (w/v) sorbitol, 0.02% (w/v) polysorbate 80 and 150 mg/ml of an active ingredient, and has the pH adjusted to 5.5 to 6.5.

[17] The pharmaceutical composition according to [16], which is for subcutaneous administration.

[18] The pharmaceutical composition according to any one of [1] to [15-1], which includes 10 mM histidine, 3.6% (w/v) sorbitol, 0.02% (w/v) polysorbate 80 and 10 mg/ml of the active ingredient, and has the pH adjusted to 5.5 to 6.5.

[19] The pharmaceutical composition according to [18], which is for intravenous administration.

[20] The pharmaceutical composition according to any one of [1] to [19], wherein the active ingredient is A10-1C04.

[21] A lyophilized preparation of a pharmaceutical composition according to any one of [1] to [20].

[22] A method for treatment or prevention of IL-33-associated diseases which includes administering a pharmaceutical composition comprising human anti-IL-33 monoclonal antibody as an active ingredient to a patient who requires it, wherein:

the combination of amino acid sequences in the heavy chain complementarity determining region 1 (H1), the heavy chain complementarity determining region 2 (H2), the heavy chain complementarity determining region 3 (H3), the light chain complementarity determining region 1 (L1), the light chain complementarity determining region 2 (L2) and the light chain complementarity determining region 3 (L3) of the human anti-IL-33 monoclonal antibody is one from among C1 to C5 listed in Table 1, and
the pharmaceutical composition contains substantially no sodium chloride or contains sodium chloride at less than 30 mM.

[23] The method according to [22], wherein the combination of amino acid sequences of the human anti-IL-33 monoclonal antibody heavy chain variable region and light chain variable region is any one from among V1 to V5 listed in Table 2.

[24] The method according to [22] or [23], wherein the human anti-IL-33 monoclonal antibody is A10-1C04, A23-1A05, A25-2C02, A25-3H04 or A26-1F02.

[25] The method according to any one of [22] to [24], wherein the sodium chloride concentration of the pharmaceutical composition is 10 mM or lower.

[26] The method according to any one of [22] to [25], wherein the pharmaceutical composition contains substantially no sodium chloride.

[27] The method according to any one of [22] to [26], wherein the pharmaceutical composition is adjusted to have a pH of higher than 4 and lower than 8.

[28] The method according to any one of [22] to [27], wherein the pharmaceutical composition is adjusted to have a pH of 5 to 7.

[29] The method according to any one of [22] to [28], wherein the pharmaceutical composition has its pH adjusted by an acetate, histidine or phosphate buffer.

[30] The method according to any one of [22] to [29], wherein the pharmaceutical composition has its pH adjusted by histidine.

[31] The pharmaceutical composition according to any one of [22] to [30], wherein the concentration of the active ingredient is less than 175 mg/ml.

[32] The method according to any one of [22] to [31], wherein the concentration of the active ingredient is 150 mg/ml or lower.

[33] The method according to any one of [22] to [32], wherein the pharmaceutical composition is one or more selected from the group consisting of sorbitol, sucrose, trehalose and mannitol.

[34] The method according to any one of [22] to [33], wherein the pharmaceutical composition contains 3 to 5% (w/v) sorbitol.

[35] The method according to any one of [22] to [34], wherein the pharmaceutical composition contains a surfactant.

[36] The method according to [35], wherein the surfactant is polysorbate 20, polysorbate 80 or poloxamer 188.

[37] The method according to any one of [22] to [36], which includes 10 mM histidine, 4% (w/v) sorbitol, 0.02% (w/v) polysorbate 80 and 150 mg/ml of an active ingredient, and has the pH adjusted to 5.5 to 6.5.

[38] The method according to [37], wherein the administration is subcutaneous administration.

[39] The method according to any one of [22] to [36], which includes 10 mM histidine, 3.6% (w/v) sorbitol, 0.02% (w/v) polysorbate 80 and 10 mg/ml of an active ingredient, and has the pH adjusted to 5.5 to 6.5.

[40] The method according to [39], wherein the administration is intravenous administration.

[41] The method according to any one of [22] to [40], wherein the active ingredient is A10-1C04.

[42] A pharmaceutical composition for use in treatment or prevention of IL-33-associated diseases, wherein:

the pharmaceutical composition comprises human anti-IL-33 monoclonal antibody as an active ingredient,
the combination of amino acid sequences in the heavy chain complementarity determining region 1 (H1), the heavy chain complementarity determining region 2 (H2), the heavy chain complementarity determining region 3 (H3), the light chain complementarity determining region 1 (L1), the light chain complementarity determining region 2 (L2) and the light chain complementarity determining region 3 (L3) of the human anti-IL-33 monoclonal antibody is one from among C1 to C5 listed in Table 1, and
the pharmaceutical composition contains substantially no sodium chloride or contains sodium chloride at less than 30 mM.

[43] The use of a pharmaceutical composition for treatment or prevention of IL-33-associated diseases, wherein:

the pharmaceutical composition is one comprising human anti-IL-33 monoclonal antibody and containing substantially no sodium chloride or containing sodium chloride at less than 30 mM, and
the combination of amino acid sequences in the heavy chain complementarity determining region 1 (H1), the heavy chain complementarity determining region 2 (H2), the heavy chain complementarity determining region 3 (H3), the light chain complementarity determining region 1 (L1), the light chain complementarity determining region 2 (L2) and the light chain complementarity determining region 3 (L3) of the human anti-IL-33 monoclonal antibody is one from among C1 to C5 listed in Table 1.

[44] The pharmaceutical composition according to [12-1], wherein the disaccharide is a saccharide selected from the group consisting of sucrose and trehalose, and the sugar alcohol is a saccharide selected from the group consisting of sorbitol and mannitol.

[45] The pharmaceutical composition according to [12-1] or [44], wherein the disaccharide is sucrose and the sugar alcohol is sorbitol.

[46] The pharmaceutical composition according to any one of [1] to [12], which contains at least one polyol having a solubility of 100 g/100 g or greater in water at 20°C.

[47] The pharmaceutical composition according to [46], wherein the polyol having a solubility of 100 g/100 g or greater in water at 20°C is a saccharide having a solubility of 100 g/100 g or greater in water at 20°C.

[48] The pharmaceutical composition according to [47], wherein the saccharide having a solubility of 100 g/100 g or greater in water at 20°C is a saccharide selected from among sorbitol and sucrose.

[49] The pharmaceutical composition according to [15], wherein the nonionic surfactant is polysorbate 20, polysorbate 80 or poloxamer 188.

[50] A pharmaceutical composition comprising human anti-IL-33 monoclonal antibody as an active ingredient, wherein:

the combination of amino acid sequences in the heavy chain complementarity determining region 1 (H1), the heavy chain complementarity determining region 2 (H2), the heavy chain complementarity determining region 3 (H3), the light chain complementarity determining region 1 (L1), the light chain complementarity determining region 2 (L2) and the light chain complementarity determining region 3 (L3) of the human anti-IL-33 monoclonal antibody is one from among C1 to C5 listed in Table 1, and
the pharmaceutical composition contains a buffering agent, a nonionic surfactant and a polyol.

[51] The pharmaceutical composition according to [50], wherein the polyol is a polyol having a solubility of 100 g/100 g or greater in water at 20°C.
[52] The pharmaceutical composition according to [50] or [51], which contains substantially no sodium chloride, or contains sodium chloride at less than 30 mM.

[ADVANTAGEOUS EFFECTS OF INVENTION]

Since the pharmaceutical composition containing a human anti-IL-33 monoclonal antibody (A10-1C04, A23-1A05, A25-2C02, A25-3H04 or A26-1F02) of the invention (or a pharmaceutical composition used for the invention) has reduced clouding, it has excellent safety and efficacy. The pharmaceutical composition of the invention can be stably stored for prolonged periods in a solution state without significant variation in the pH, and is therefore suitable for storage and use of formulations.

BRIEF DESCRIPTION OF DRAWINGS

Fig. 1 shows clouding of an A10-1C04 antibody P7N formulation.
Fig. 2 shows the clouding-inhibiting effect by sugar addition. Clouding was improved with A10-1C04 antibody formulations with sorbitol addition (A5S, H6S, P6S, P7S).

DESCRIPTION OF EMBODIMENTS

The terms used herein will now be explained for clearer understanding of the invention.

[Pharmaceutical composition]

As used herein, "pharmaceutical composition" refers to a composition prepared so as to be administrable to an animal such as a human. The term "pharmaceutical composition" may therefore refer to a formulation in dosage form.

[Substantially]

As used herein, "contains substantially no sodium chloride" means that no sodium chloride is added to the pharmaceutical composition of the invention, and that it does not contain sodium chloride in an amount that causes clouding of the pharmaceutical composition of the invention.

[Surfactant]

As used herein, "surfactant" is a general term for a substance having in the molecule a portion with affinity for water (hydrophilic group) and a portion with affinity for oils (lipophilic group or hydrophobic group). Any surfactant may be used that is commonly employed in antibody formulations, with polysorbate 20, polysorbate 80 and poloxamer 188 being examples.

[Antibody]

The term "antibody" used herein is used in its widest sense to include human antibodies, humanized antibodies and antibodies from non-human species, and either monoclonal antibodies or polyclonal antibodies. The term "antibody" used herein may also refer to a multispecific antibody (such as a bispecific antibody) or an antibody-drug conjugate (ADC), or an antigen-binding fragment such as dAbs, scFv, Fab, F(ab)'2 or Fab'.

[Monoclonal antibody]

The term "monoclonal antibody", as used herein, refers to an antibody consisting of a group of substantially uniform antibodies, i.e. where the individual antibodies of the group are identical except for minor differences such as sugar chain or amino acid modifications. Monoclonal antibodies generally bind to a single epitope on an antigen, in contrast to polyclonal antibodies which include different antibodies. The adjective "monoclonal" indicates the feature of an antibody that can be obtained from a group of substantially uniform antibodies, and is not to be interpreted as requiring preparation of the antibody by a specific method. For example, the monoclonal antibody used for the invention may be produced by the hybridoma method first described in Kohler et al., Nature, 256:495(1975), or by a recombinant DNA method (see USP 4,816,567, for example). A "monoclonal antibody" may also be one isolated from a phage antibody library using the techniques described in Clackson et al., Nature, 352: 624-628(1991) and Marks et al., J. Mol. Biol., 222: 581-597 (1991), for example.

[Five types of human anti-IL-33 monoclonal antibodies]

The five types of human anti-IL-33 monoclonal antibodies to be used for the invention are human anti-IL-33 monoclonal antibodies of the 5 clones A10-1C04, A23-1A05, A25-2C02, A25-3H04 and A26-1F02 disclosed in International Patent Publication No. 2015/099175, the human anti-IL-33 monoclonal antibodies having heavy chains and light chains with the following amino acid sequences.

(a) A10-1C04:

- Heavy chain:

EVQLLESGGGLVQPGGSLRLSCAASGFTFSDYYMNWVRQAPGKGLEWVSSISRYSSYIY YADSVKGRFTISRDNSKNTLYLQMNSLRAEDTAVYYCARDIGGMDVWGQGTLVTVSS ASTKGPSVFPLAPSSKSTSGGTAALGCLVKDYFPEPVTVSWNSGALTSGVHTFPAVLQSS GLYSLSSVVTVPSSSLGTQTYICNVNHKPSNTKVDKKVEPKSCDKTHTCPPCPAPELLGG PSVFLFPPKPKDTLMISRTPEVTCVVVDVSHEDPEVKFNWYVDGVEVHNAKTKPREEQY NSTYRVVSVLTVLHQDWLNGKEYKCKVSNKALPAPIEKTISKAKGQPREPQVYTLPPSR DELTKNQVSLTCLVKGFYPSDIAVEWESNGQPENNYKTTPPVLDSDGSFFLYSKLTVDK SRWQQGNVFSCSVMHEALHNHYTQKSLSLSPGK (SEQ ID NO: 1);

and

- Light chain:

QSVLTQPPSASGTPGQRVTISCTGSSSNIGAVYDVHWYQQLPGTAPKLLIYRNNQRPSGV PDRFSGSKSGTSASLAISGLRSEDEADYYCQTYDSSRWVFGGGTKLTVLGQPKAAPSVT LFPPSSEELQANKATLVCLISDFYPGAVTVAWKADSSPVKAGVETTTPSKQSNNKYAAS SYLSLTPEQWKSHRSYSCQVTHEGSTVEKTVAPTECS (SEQ ID NO: 2)

(b) A23-1A05:

- Heavy chain:

EVQLLESGGGLVQPGGSLRLSCAASGFTFSNYYMHWVRQAPGKGLEWVSSISARSRYH YYADSVKGRFTISRDNSKNTLYLQMNSLRAEDTAVYYCARLATRHNAFDIWGQGTLVT VSSASTKGPSVFPLAPSSKSTSGGTAALGCLVKDYFPEPVTVSWNSGALTSGVHTFPAVL QSSGLYSLSSVVTVPSSSLGTQTYICNVNHKPSNTKVDKKVEPKSCDKTHTCPPCPAPEL LGGPSVFLFPPKPKDTLMISRTPEVTCVVVDVSHEDPEVKFNWYVDGVEVHNAKTKPRE EQYNSTYRVVSVLTVLHQDWLNGKEYKCKVSNKALPAPIEKTISKAKGQPREPQVYTLP PSRDELTKNQVSLTCLVKGFYPSDIAVEWESNGQPENNYKTTPPVLDSDGSFFLYSKLTV DKSRWQQGNVFSCSVMHEALHNHYTQKSLSLSPGK (SEQ ID NO: 3);

and

• Light chain:

QSVLTQPPSASGTPGQRVTISCSGSSSNIGNNAVSWYQQLPGTAPKLLIYASNMRVIGVP DRFSGSKSGTSASLAISGLRSEDEADYYCGAWDDSQKALVFGGGTKLTVLGQPKAAPS VTLFPPSSEELQANKATLVCLISDFYPGAVTVAWKADSSPVKAGVETTTPSKQSNNKYA ASSYLSLTPEQWKSHRSYSCQVTHEGSTVEKTVAPTECS (SEQ ID NO: 4)

(c) A25-2C02:

• Heavy chain:

EVQLLESGGGLVQPGGSLRLSCAASGFTFSNYYMHWVRQAPGKGLEWVSSISARSSYIY YADSVKGRFTISRDNSKNTLYLQMNSLRAEDTAVYYCARLATRNNAFDIWGQGTLVTV SSASTKGPSVFPLAPSSKSTSGGTAALGCLVKDYFPEPVTVSWNSGALTSGVHTFPAVLQ SSGLYSLSSVVTVPSSSLGTQTYICNVNHKPSNTKVDKKVEPKSCDKTHTCPPCPAPELL GGPSVFLFPPKPKDTLMISRTPEVTCVVVDVSHEDPEVKFNWYVDGVEVHNAKTKPREE QYNSTYRVVSVLTVLHQDWLNGKEYKCKVSNKALPAPIEKTISKAKGQPREPQVYTLPP SRDELTKNQVSLTCLVKGFYPSDIAVEWESNGQPENNYKTTPPVLDSDGSFFLYSKLTV DKSRWQQGNVFSCSVMHEALHNHYTQKSLSLSPGK (SEQ ID NO: 5);

and
• Light chain:

QSVLTQPPSASGTPGQRVTISCSGSSSNIGRNAVNWYQQLPGTAPKLLIYASNMRVSGVP DRFSGSKSGTSASLAISGLRSEDEADYYCWAWDDSQKVGVFGGGTKLTVLGQPKAAPS VTLFPPSSEELQANKATLVCLISDFYPGAVTVAWKADSSPVKAGVETTTPSKQSNNKYA ASSYLSLTPEQWKSHRSYSCQVTHEGSTVEKTVAPTECS (SEQ ID NO: 6)

(d) A25-3H04:

- Heavy chain:

EVQLLESGGGLVQPGGSLRLSCAASGFTFSRYYMHWVRQAPGKGLEWVSSISAQSSHIY YADSVEGRFTISRDNSKNTLYLQMNSLRAEDTAVYYCARLATRQNAFDIWGQGTLVTV SSASTKGPSVFPLAPSSKSTSGGTAALGCLVKDYFPEPVTVSWNSGALTSGVHTFPAVLQ SSGLYSLSSVVTVPSSSLGTQTYICNVNHKPSNTKVDKKVEPKSCDKTHTCPPCPAPELL GGPSVFLFPPKPKDTLMISRTPEVTCVVVDVSHEDPEVKFNWYVDGVEVHNAKTKPREE QYNSTYRVVSVLTVLHQDWLNGKEYKCKVSNKALPAPIEKTISKAKGQPREPQVYTLPP SRDELTKNQVSLTCLVKGFYPSDIAVEWESNGQPENNYKTTPPVLDSDGSFFLYSKLTV DKSRWQQGNVFSCSVMHEALHNHYTQKSLSLSPGK (SEQ ID NO: 7);

and
- Light chain:

QSVLTQPPSASGTPGQRVTISCSGSSSNIGRNAVNWYQQLPGTAPKLLIYASNMRRSGVP DRFSGSKSGTSASLAISGLRSEDEADYYCSAWDDSQKVVVFGGGTKLTVLGQPKAAPSV TLFPPSSEELQANKATLVCLISDFYPGAVTVAWKADSSPVKAGVETTTPSKQSNNKYAA SSYLSLTPEQWKSHRSYSCQVTHEGSTVEKTVAPTECS (SEQ ID NO: 8)

(e) A26-1F02:

- Heavy chain:

EVQLLESGGGLVQPGGSLRLSCAASGFTFSNYYMHWVRQAPGKGLEWVSSISARSSYL YYADSVKGRFTISRDNSKNTLYLQMNSLRAEDTAVYYCARLATRHVAFDIWGQGTLVT VSSASTKGPSVFPLAPSSKSTSGGTAALGCLVKDYFPEPVTVSWNSGALTSGVHTFPAVL QSSGLYSLSSVVTVPSSSLGTQTYICNVNHKPSNTKVDKKVEPKSCDKTHTCPPCPAPEL LGGPSVFLFPPKPKDTLMISRTPEVTCVVVDVSHEDPEVKFNWYVDGVEVHNAKTKPRE EQYNSTYRVVSVLTVLHQDWLNGKEYKCKVSNKALPAPIEKTISKAKGQPREPQVYTLP PSRDELTKNQVSLTCLVKGFYPSDIAVEWESNGQPENNYKTTPPVLDSDGSFFLYSKLTV DKSRWQQGNVFSCSVMHEALHNHYTQKSLSLSPGK (SEQ ID NO: 9);

and
- Light chain:

QSVLTQPPSASGTPGQRVTISCSGSSSNIGNNAVNWYQQLPGTAPKLLIYASNMRRPGVP
DRFSGSKSGTSASLAISGLRSEDEADYYCEAWDDSQKAVVFGGGTKLTVLGQPKAAPS
VTLFPPSSEELQANKATLVCLISDFYPGAVTVAWKADSSPVKAGVETTTPSKQSNNKYA
ASSYLSLTPEQWKSHRSYSCQVTHEGSTVEKTVAPTECS (SEQ ID NO: 10)

[Complementarity Determining Region (CDR)]

The complementarity determining region (CDR), as used herein, comprises the amino acid residues of the antibody that are involved in antigen binding. A CDR is generally referred to as a "hypervariable region", and it has a unique amino acid sequence for each antibody species and is determined by the method of Kabat et al. (Kabat et al., Sequences of Proteins of Immunological Interest, 5th Ed.Public Health Service, National Institutes of Health, Bethesda, Md. (1991)). There are 3 CDRs on the antibody light chain (L1, L2, L3) and 3 on the antibody heavy chain (H1, H2, H3), and the respective CDRs of the 5 clones A10-1C04, A23-1A05, A25-2C02, A25-3H04 and A26-1F02 are as follows.

(a) A10-1C04:

H1:DYYMN (SEQ ID NO: 11)
H2:SISRYSSYIYYADSVKG (SEQ ID NO: 12)
H3:DIGGMDV (SEQ ID NO: 13)
L1:TGSSSNIGAVYDVH (SEQ ID NO: 14)
L2:RNNQRPS (SEQ ID NO: 15)
L3:QTYDSSRWV (SEQ ID NO: 16)

(b) A23-1A05:

H1:NYYMH (SEQ ID NO: 17)
H2:SISARSRYHYYADSVKG (SEQ ID NO: 18)
H3:LATRHNAFDI (SEQ ID NO: 19)
L1:SGSSSNIGNNAVS (SEQ ID NO: 20)
L2:ASNMRVI (SEQ ID NO: 21)
L3:GAWDDSQKALV (SEQ ID NO: 22)

(c) A25-2C02:

H1:NYYMH (SEQ ID NO: 17)
H2:SISARSSYIYYADSVKG (SEQ ID NO: 23)
H3:LATRNNAFDI (SEQ ID NO: 24)
L1:SGSSSNIGRNAVN (SEQ ID NO: 25)
L2:ASNMRVS (SEQ ID NO: 26)
L3:WAWDDSQKVGV (SEQ ID NO: 27)

(d) A25-3H04:

H1:RYYMH (SEQ ID NO: 28)
H2:SISAQSSHIYYADSVEG (SEQ ID NO: 29)
H3:LATRQNAFDI (SEQ ID NO: 30)
L1:SGSSSNIGRNAVN (SEQ ID NO: 25)

L2:ASNMRRS (SEQ ID NO: 31)
L3:SAWDDSQKVVV (SEQ ID NO: 32)

(e) A26-1F02:

H1:NYYMH (SEQ ID NO: 17)
H2:SISARSSYLYYADSVKG (SEQ ID NO: 33)
H3:LATRHVAFDI (SEQ ID NO: 34)
L1:SGSSSNIGNNAVN (SEQ ID NO: 35)
L2:ASNMRRP (SEQ ID NO: 36)
L3:EAWDDSQKAVV (SEQ ID NO: 37)

[Variable region]

The term "variable region" as used herein refers to the portion of a monoclonal antibody other than the constant region, being the portion that is involved in antigen binding and that determines the specificity of an antibody, which varies depending on the type of antigen. The variable region includes a heavy chain variable region and a light chain variable region, the heavy chain variable regions and light chain variable regions of the 5 clones A10-1C04, A23-1A05, A25-2C02, A25-3H04 and A26-1F02 being as follows.

(a) A10-1C04:

• Heavy chain variable region:

EVQLLESGGGLVQPGGSLRLSCAASGFTFSDYYMNWVRQAPGKGLEWVSSISRYSSYIY YADSVKGRFTISRDNSKNTLYLQMNSLRAEDTAVYYCARDIGGMDVWGQGTLVTVSS (SEQ ID NO: 38)

• Light chain variable region:

QSVLTQPPSASGTPGQRVTISCTGSSSNIGAVYDVHWYQQLPGTAPKLLIYRNNQRPSGV PDRFSGSKSGTSASLAISGLRSEDEADYYCQTYDSSRWVFGGGTKLTVLG (SEQ ID NO: 39)

(b) A23-1A05:

• Heavy chain variable region:

EVQLLESGGGLVQPGGSLRLSCAASGFTFSNYYMHWVRQAPGKGLEWVSSISARSRYH YYADSVKGRFTISRDNSKNTLYLQMNSLRAEDTAVYYCARLATRHNAFDIWGQGTLVT VSS (SEQ ID NO: 40)

• Light chain variable region:

QSVLTQPPSASGTPGQRVTISCSGSSSNIGNNAVSWYQQLPGTAPKLLIYASNMRVIGVP DRFSGSKSGTSASLAISGLRSEDEADYYCGAWDDSQKALVFGGGTKLTVLG (SEQ ID NO: 41)

(c) A25-2C02:

• Heavy chain variable region:

EVQLLESGGGLVQPGGSLRLSCAASGFTFSNYYMHWVRQAPGKGLEWVSSISARSSYIY YADSVKGRFTISRDNSKNTLYLQMNSLRAEDTAVYYCARLATRNNAFDIWGQGTLVTV SS (SEQ ID NO: 42)

• Light chain variable region:

QSVLTQPPSASGTPGQRVTISCSGSSSNIGRNAVNWYQQLPGTAPKLLIYASNMRVSGVP DRFSGSKSGTSASLAISGLRSEDEADYYCWAWDDSQKVGVFGGGTKLTVLG (SEQ ID NO: 43)

(d) A25-3H04:

• Heavy chain variable region:

EVQLLESGGGLVQPGGSLRLSCAASGFTFSRYYMHWVRQAPGKGLEWVSSISAQSSHIY YADSVEGRFTISRDNSKNTLYLQMNSLRAEDTAVYYCARLATRQNAFDIWGQGTLVTV SS (SEQ ID NO: 44)

• Light chain variable region:

QSVLTQPPSASGTPGQRVTISCSGSSSNIGRNAVNWYQQLPGTAPKLLIYASNMRRSGVP DRFSGSKSGTSASLAISGLRSEDEADYYCSAWDDSQKVVVFGGGTKLTVLG (SEQ ID NO: 45)

(e) A26-1F02:

• Heavy chain variable region:

EVQLLESGGGLVQPGGSLRLSCAASGFTFSNYYMHWVRQAPGKGLEWVSSISARSSYL YYADSVKGRFTISRDNSKNTLYLQMNSLRAEDTAVYYCARLATRHVAFDIWGQGTLVT VSS (SEQ ID NO: 46)

- Light chain variable region:

QSVLTQPPSASGTPGQRVTISCSGSSSNIGNNAVNWYQQLPGTAPKLLIYASNMRRPGVP DRFSGSKSGTSASLAISGLRSEDEADYYCEAWDDSQKAVVFGGGTKLTVLG (SEQ ID NO: 47)

"Stability"

The term "stability", as used herein, means that the antibody-containing medicinal composition essentially retains its properties (such as physical properties, chemical properties and/or biological activity) even after storage. Various analysis techniques for measuring the stability of proteins such as antibodies are available in the technical field, and are explained in overview in Peptide and Protein Drug Delivery, 247-301, Vincent Lee Ed., Marcel Dekker, Inc., New York, N.Y., Pubs. (1991) and Jones, A. Adv. Drug Delivery Rev. 10:29-90 (1993). The stability of the antibody-containing medicinal composition can be evaluated at a selected temperature for a selected time. A "stable" antibody-containing medicinal composition is an antibody-containing medicinal composition that exhibits no significant changes when left to stand at refrigerating temperature (2 to 8°C) for at least 1 month, 3 months, 6 months or 12 months, and preferably 2 years or more preferably 3 years, at room temperature (23 to 27°C) for at least 3 months, preferably 6 months and more preferably 1 year, or under stress conditions (about 40°C or about 50°C) for at least one week, 2 weeks or one month, and preferably 3 months or more preferably 6 months. Various stability criteria, including abnormalities in visual examination (such as clouding), pH, viscosity, antibody binding to antigen, antibody inhibiting activity against antigen molecules (such as inhibition of IL-6 induction by IL-33), antibody effector function and antibody decomposition, can be used as indicators.

"Effector function"

The "effector function" of an antibody is the bioactivity exhibited due to the Fc region of the antibody (the Fc region of the natural sequence or the Fc region of a mutated amino acid sequence). Examples of antibody effector functions include C1q bonding, complement-dependent cytotoxicity, Fc receptor binding, antibody-dependent cell mediated cytotoxicity (ADCC), phagocytosis, and downregulation of cell surface receptors (such as B cell receptor, BCR).

[Clouding]

As used herein, "clouding" means a state of white cloudiness determined by visual examination of color and/or transparency (turbidity). Clouding can be analyzed by measurement of microparticles using a flow cytometric image analyzer or a particle counter, measurement of interaction parameters by dynamic light scattering (DLS) (hereunder referred to simply as "interaction parameters" or "Kd value"), or measurement of turbidity (absorbance at 650 nm (OD650)). The measured values correlating to "clouding" for an antibody-containing pharmaceutical composition are an OD650 of 0.009, 0.010, 0.011, 0.012, 0.013 or 0.014 or greater, a Kd value of 0, -1, -2, -3 or -4 mL/g or lower, and/or 500, 750, 1000, 1250 or 1500/mL or more particles of 1.5 $\mu$m or greater determined using a particle counter.

[Lyophilized preparation]

A "lyophilized preparation", as used herein, is a pharmaceutical composition dried with virtually no water (for example, freeze-dried). Lyophilization techniques for antibodies are well known in the technical field and are described in Rey & May (2004) Freeze-Drying/Lyophilization of Pharmaceutical & Biological Products ISBN 0824748689, for example.

[IL-33]

IL-33 is a cytokine belonging to the IL-1 family, being also known as NF-HEV. When released from cells as a cytokine, IL-33 binds to IL-33 receptors (ST2 and IL-1RAcP), functioning to initiate intracellular signal transduction in cells expressing the IL-33 receptors. Signal transduction induced by IL-33 takes place, although not exclusively, via the NF-KB pathway and MAPKKs pathway, eventually eliciting production of various cytokines and chemokines or inflammatory mediators. Examples of cytokines elicited by IL-33 include TNF-$\alpha$, IL-1$\beta$, IL-3, IL-4, IL-5, IL-6 and IL-13, with IL-5, IL-6 and IL-13 being elicited in particular. Examples of chemokines elicited by IL-33 include CXCL2, CCL2, CCL3, CCL6, CCL17 and CCL24. Examples of inflammatory mediators elicited by IL-33 include PGD2 and LTB4. The cytokines, chemokines and inflammatory mediators elicited by IL-33 in turn elicit migration of immune system cells, production of cytokines, and inflammation via degranulation. For the purpose of the invention, so long as the "five types of human anti-IL-33 monoclonal antibodies" bind to inhibit at least one function among the functions mentioned above, the IL-33 may be full length IL-33 or mature IL-33, or it may be a homologous derivative or mutant. It may also be human IL-33 or IL-33 derived from another organism.

[Pharmaceutically acceptable]

The term "pharmaceutically acceptable", as used herein, means no interference of the efficacy of biological activity of (optionally multiple) active ingredients, and no toxicity.

[Isotonicity]

An "isotonic" formulation, for the purpose of the present specification, is one having essentially the same osmotic pressure as human blood. An isotonic pharmaceutical composition generally has an osmotic pressure ratio of about 0.9 to 1.2, based on blood. The osmotic pressure can be measured using a vapor pressure-type or ice-freezing osmometer, for example.

[pH drift]

The term "pH drift", as used herein, means a change in the pH value of the pharmaceutical composition of the invention before and after storage or treatment such as concentration.
An embodiment of the invention will now be explained. The following embodiment is an example for illustration of the invention, with the understanding that the invention is not limited to this embodiment.
The human anti-IL-33 monoclonal antibody of the invention can be produced by a publicly known technique, such as the method described in PTL 1, for example.
The monoclonal antibody produced in the manner described above can be formulated into a desired composition by a method such as dialysis or ultrafiltration, or ammonium sulfate precipitation. It may also be prepared as a solution containing a desired buffering agent, with subsequent addition of a saccharide or surfactant to obtain a formulation.
The present inventors have found that when the five types of human anti-IL-33 monoclonal antibodies are prepared as solutions, they undergo partial aggregation and exhibit clouding. Clouding occurs due to formation of microparticles consisting of antibody-containing aggregates, and when such a solution is prepared as a pharmaceutical composition it can result in lower bioactivity of the antibody or impaired pharmacokinetics due to anti-drug antibodies (ADA) produced by the highly immunogenic aggregates, as well as potentially eliciting inflammation by the aggregates themselves. It is therefore necessary to reduce clouding. The present invention relates to a pharmaceutical composition that has reduced clouding. The pharmaceutical composition of the invention contains five types of human anti-IL-33 monoclonal antibodies as active ingredients, and may also contain a salt, buffering agent, surfactant, saccharide and the like.

[Salt concentration]

The present inventors have found that salts are the major cause of clouding in the five types of human anti-IL-33 monoclonal antibodies. The pharmaceutical composition of the invention therefore preferably has a low salt concentration, which is preferably 50 mM or lower, 40 mM or lower, 30 mM or lower, 25 mM or lower, 20 mM or lower, 15 mM or lower, 10 mM or lower, 5 mM or lower, 3 mM or lower, 2 mM or lower or 1 mM or lower, and more preferably it contains substantially no salts. The low pharmaceutical composition of the invention is preferably lower than 50 mM, lower than 40 mM, lower than 30 mM, lower than 25 mM, lower than 20 mM, lower than 15 mM, lower than 10 mM, lower than 5 mM, lower than 3 mM, lower than 2 mM or lower than 1 mM, and more preferably it contains substantially no salts. Examples of salts to have low concentration or to be substantially absent in the pharmaceutical composition of the invention are inorganic salts and organic salts. Examples of inorganic salts include sodium chloride, potassium chloride, magnesium chloride, calcium chloride, sodium sulfate, potassium sulfate, magnesium sulfate and calcium sulfate, with sodium chloride being preferred.

[Buffering agent and pH]

The pharmaceutical composition of the invention has its pH adjusted with a buffering agent. The buffering agent to be used in the pharmaceutical composition is not restricted, and may be a gluconate, histidine, citrate, phosphate [such as sodium or potassium], succinate [such as sodium], acetate, trishydroxymethylaminomethane, glycine or arginine, or a combination of these, which may be used as appropriate depending on the target pH value for adjustment. The pharmaceutical composition of the invention preferably includes an acetate, histidine or a phosphate as a buffering agent, and more preferably it includes histidine as a buffering agent. The buffering agent concentration is not particularly restricted so long as it is pharmaceutically acceptable, but it is preferably 1 mM to 150 mM, more preferably 5 mM to 100 mM and even more preferably 10 mM to 50 mM. The buffering agent concentration is preferably about 1 mM, about 5 mM, about 10 mM, about 15 mM, about 20 mM, about 25 mM, about 30 mM, about 35 mM, about 40 mM, about 45 mM or about 50 mM. From the viewpoint of using a salt (especially inorganic salt) buffering agent, the concentration is preferably less than 30 mM, and especially 10 mM or lower.

It is particularly useful to use histidine (for example, 5 mM to 50 mM, or 10 mM to 50 mM, about 5 mM, about 10 mM, about 15 mM, about 20 mM, about 25 mM, about 30 mM, about 35 mM, about 40 mM, about 45 mM or about 50 mM) as the buffering agent in the pharmaceutical composition of the invention. According to one embodiment, a stable pharmaceutical composition contains 5 mM to 20 mM histidine. The pH of the pharmaceutical composition may be in the range of 4.0 to 8.0, with common pH values in the range of 4.5 to 7.5 being 5.0 to 7.0 or 5.2 to 6.8, such as about 4.2, about 4.3, about 4.4, about 4.5, about 4.6, about 4.7, about 4.8, about 4.9, about 5.0, about 5.1, about 5.2, about 5.3, about 5.4, about 5.5, about 5.6, about 5.7, about 5.8, about 5.9, about 6.0, about 6.1, about 6.2, about 6.3, about 6.4, about 6.5, about 6.6, about 6.7, about 6.8, about 6.9, about 7.0, about 7.1, about 7.2, about 7.3, about 7.4, about 7.5, about 7.6, about 7.7 or about 7.8. The pharmaceutical composition of the invention has increased turbidity upon prolonged storage at pH 4 or pH 8. The pharmaceutical composition of the invention has significant pH drift upon prolonged storage at pH 4 or pH 8. According to one embodiment, therefore, the pH of a stable antibody-containing pharmaceutical composition is higher than 4 and lower than 8, preferably between 5 and 7, even more preferably between 5.5 and 6.5 and most preferably 6.0.

[Surfactant]

The pharmaceutical composition of the invention preferably includes a surfactant. Surfactants that are suitable for the pharmaceutical composition include, but are not limited to, nonionic surfactants, ionic surfactants and zwitterionic surfactants, as well as combinations of these. Common surfactants for the invention include, but are not limited to, sorbitan fatty acid esters (such as sorbitan monocaprylate, sorbitan monolaurate and sorbitan monopalmitate), sorbitan trioleate, glycerin fatty acid esters (such as glycerin monocaprylate, glycerin monomyristate and glycerin monostearate), polyglycerin fatty acid esters (such as decaglyceryl monostearate, decaglyceryl distearate and decaglyceryl monolinolate), polyoxyethylene sorbitan fatty acid esters (such as polyoxyethylene sorbitan monolaurate, polyoxyethylene sorbitan monooleate, polyoxyethylene sorbitan monostearate, polyoxyethylene sorbitan monopalmitate, polyoxyethylene sorbitan trioleate and polyoxyethylene sorbitan tristearate), polyoxyethylene sorbitol fatty acid esters (such as polyoxyethylene sorbitol tetrastearate and polyoxyethylene sorbitol tetraoleate), polyoxyethylene glycerin fatty acid esters (such as polyoxyethylene glyceryl monostearate), polyethylene glycol fatty acid esters (such as polyethyleneglycol distearate), polyoxyethylene alkyl ethers (such as polyoxyethylene lauryl ether), polyoxyethylene polyoxypropylene alkyl ethers (such as polyoxyethylene polyoxypropylene glycol, polyoxyethylene polyoxypropylene propyl ether and polyoxyethylene polyoxypropylene cetyl ether), polyoxyethylene alkylphenyl ethers (such as polyoxyethylene nonylphenyl ether), polyoxyethylene hardened castor oils (such as polyoxyethylene castor oil and polyoxyethylene hardened castor oil), polyoxyethylene beeswax derivatives (such as polyoxyethylene sorbitol beeswax), polyoxyethylene lanolin derivatives (such as polyoxyethylene lanolin), polyoxyethylene fatty acid amides (such as amide polyoxyethylene stearate), C10 to C18 alkylsulfuric acids (such as sodium cetylsulfate, sodium laurylsulfate and sodium oleylsulfate), polyoxyethylene C10 to C18 alkylether sulfates having an average of 2 to 4 mol of ethylene oxide units (such as sodium polyoxyethylene laurylsulfate) and C1 to C18 alkylsulfosuccinic acid ester salts (such as sodium laurylsulfosuccinate ester), as well as natural surfactants such as lecithin, glycerophospholipids and sphingophospholipids (such as sphingomyelins), and C12 to C18 fatty acid sucrose esters. The pharmaceutical composition of the invention may include one or more of these surfactants. Preferred surfactants are nonionic surfactants (such as sorbitan fatty acid esters, sorbitan trioleate, glycerin fatty acid esters, polyglycerin fatty acid esters, polyoxyethylene sorbitan fatty acid esters, polyoxyethylene sorbitol fatty acid esters, polyoxyethylene glycerin fatty acid esters, polyoxyethylene alkyl ethers, polyoxyethylene polyoxypropylene alkyl ethers, polyoxyethylene alkylphenyl ethers, polyoxyethylene hardened castor oil, polyoxyethylene beeswax derivatives, polyoxyethylene lanolin derivatives and polyoxyethylene fatty acid amides), and more preferably polyoxyethylene alkyl ethers (such as poloxamer 188) or polyoxyethylene sorbitan fatty acid esters, such as polysorbate 20, 40, 60 or 80. The concentration of the surfactant may be any concentration that is commonly employed in the technical field, examples of which are concentrations of about 0.01% (w/v) to about 0.1% (w/v), such as about 0.01% (w/v) to about 0.04% (w/v), about 0.01% (w/v), about 0.02% (w/v), about 0.04% (w/v), about 0.06% (w/v), about 0.08% (w/v) or about 0.1% (w/v). Polysorbate 80 (Tween80) is especially useful among these surfactants. According to one embodiment, the stable pharmaceutical composition includes about 0.02% (w/v) polysorbate 80. According to another embodiment, the stable pharmaceutical composition includes about 0.02% (w/v) polysorbate 20.

[Polyol]

The pharmaceutical composition of the invention preferably includes a polyol, as addition of a polyol can adjust the osmotic pressure of the pharmaceutical composition and inhibit formation of aggregates.

The polyol in the pharmaceutical composition of the invention is not particularly restricted so long as it is pharmaceutically acceptable, but it is preferably a polyol that dissolves at 100 g or greater in 100 g of water at 20°C (that is, a polyol having solubility of 100 g/100 g or greater in water at 20°C).

A polyol is a polyhydric alcohol, which may be any molecule having two or more alcoholic hydroxyl groups, examples of which include glycerol (glycerin), propylene glycol, polyethylene glycol (PEG) and saccharides, with saccharides being preferred.

Saccharides suitable for the pharmaceutical composition of the invention are not restricted and may be compounds with the formula $(CH_2O)n$ including monosaccharides, disaccharides, trisaccharides, other polysaccharides, sugar alcohols, reducing sugars and non-reducing sugars, as well as their derivatives. Examples of saccharides include monosaccharides such as glucose, fructose and galactose, disaccharides such as sucrose, trehalose, lactose, maltose, lactulose, maltulose, isomaltulose and melibiose, trisaccharides such as melezitose, raffinose and maltotriose, other polysaccharides such as stachyose and dextran, and sugar alcohols such as sorbitol, mannitol, erythritol, maltitol, lactitol, arabitol and xylitol. Reducing sugars among monosaccharides, disaccharides and trisaccharides include glucose, fructose, lactose, maltose, lactulose, maltulose, isomaltulose, melibiose, melezitose and maltotriose, while non-reducing sugars include trehalose, sucrose and raffinose. Saccharides in the pharmaceutical composition of the invention are preferably sorbitol, sucrose, trehalose or mannitol, and most preferably sorbitol or sucrose.

The concentration of saccharides in the pharmaceutical composition of the invention is not particularly restricted so long as it is pharmaceutically acceptable, but it is preferably 50 mM to 300 mM, more preferably 165 mM to 275 mM and even more preferably 200 mM to 220 mM. The saccharide concentration is preferably about 50 mM, about 55 mM, about 100 mM, about 110 mM, about 150 mM, about 165 mM, about 200 mM, about 220 mM, about 275 mM or about 300 mM. For this embodiment, a stable pharmaceutical composition includes about 3% to 5% (w/v) (165 to 275 mM) sorbitol. According to another embodiment, a stable pharmaceutical composition includes about 3.6% (w/v) or about 4% (w/v) (about 200 mM or about 220 mM, respectively) sorbitol.

The osmotic pressure ratio of the pharmaceutical composition of the invention is preferably 0.5 to 4, more preferably 0.7 to 3, even more preferably 1 or 2 and most preferably isotonic (0.9 to 1.2), and it may be about 0.9, about 1.0, about 1.1, about 1.2, about 1.3, about 1.4, about 1.5, about 1.6, about 1.7, about 1.8, about 1.9 or about 2.0, for example. The osmotic pressure of the pharmaceutical composition can be adjusted by the concentration of components other than the active ingredient, such as salts or polyols. From the viewpoint of lowering the salt concentration, the osmotic pressure of the pharmaceutical composition is preferably adjusted using a polyol.

[Human anti-IL-33 monoclonal antibodies]

The pharmaceutical composition of the invention includes a human anti-IL-33 monoclonal antibody (A10-1C04, A23-1A05, A25-2C02, A25-3H04 or A26-1F02) as an active ingredient. The concentration of the human anti-IL-33 monoclonal antibody in the pharmaceutical composition of the invention is not particularly restricted so long as it is pharmaceutically acceptable, but it is preferably 1 mg/mL to 200 mg/mL, 5 mg/mL to 175 mg/mL, 10 mg/mL to 150 mg/ml or 20 mg/mL to 150 mg/mL, and it may be about 1, about 5, about 10, about 20, about 25, about 30, about 40, about 50, about 60, about 70, about 80, about 90, about 100, about 110, about 120, about 130, about 140, about 150, about 160, about 170 or about 175 mg/mL, for example. The antibody concentration of the pharmaceutical composition of the invention will have high viscosity of 175 mg/mL or greater, and the concentration is therefore preferably less than 175 mg/mL and more preferably 150 mg/mL or lower. According to one embodiment, a stable pharmaceutical composition includes about 10 mg/mL or about 150 mg/mL of A10-1C04. According to another embodiment, a stable pharmaceutical composition includes about 10 mg/mL or about 150 mg/mL of A23-1A05. According to yet another embodiment, a stable pharmaceutical composition includes about 10 mg/mL or about 150 mg/mL of A25-2C02. According to yet another embodiment, a stable pharmaceutical composition includes about 10 mg/mL or about 150 mg/mL of A25-3H04. According to yet another embodiment, a stable pharmaceutical composition includes about 10 mg/mL or about 150 mg/mL of A26-1F02.

The pharmaceutical composition of the invention preferably has suitable viscosity to allow it to be easily administered to any patient. Low viscosity allows administration without the need for strong force, but the present inventors have found that for the pharmaceutical composition of the invention, a viscosity of above about 20 cP creates some difficulty for injection from a glass syringe at the time of administration. Therefore, the viscosity of the pharmaceutical composition of the invention is preferably 50 cP or lower, more preferably 30 cP or lower, even more preferably 20 cP or lower and most preferably 10 cP or lower. According to one embodiment, the pharmaceutical composition of the invention is about 1, about 2, about 5, about 10, about 15 or about 20 cP. The viscosity of the invention can be measured by (rotating or capillary) rheometry.

[Method of using pharmaceutical composition]

The pharmaceutical composition of the invention can be used for intervention (treatment or prevention) in patients with IL-33-associated diseases such as asthma, allergy (atopic dermatitis or pollen hypersensitivity) or endometriosis, for example. The form of administration is not particularly restricted and may be systemic administration or local administration. For example, intravenous administration, subcutaneous administration, intramuscular administration and intraperitoneal administration are possible. Since the pharmaceutical composition of the invention has a limited dose for subcutaneous administration, it preferably contains a high-concentration of human anti-IL-33 antibody, and for example, it is preferably a pharmaceutical composition containing 10 mM histidine, 4% (w/v) sorbitol, 0.02% (w/v) polysorbate 80 and 150 mg/ml of active ingredient, and with the pH adjusted to 5.5 to 6.5. The pharmaceutical composition of the invention may contain a low concentration of human anti-IL-33 antibody, for intravenous administration, for example, and it is preferably a pharmaceutical composition containing 10 mM histidine, 3.6% (w/v) sorbitol, 0.02% (w/v) polysorbate 80 and 10 mg/ml of active ingredient, and with the pH adjusted to 5.5 to 6.5, for example.

Examples of IL-33-associated diseases include, but are not limited to, asthma, atopic dermatitis, hives, pollen hypersensitivity, anaphylactic shock, eosinophilic sinusitis, eosinophilia syndrome, Churg-Strauss syndrome, allergenicity encephalomyelitis, polymyalgia rheumatica, rheumatic heart disease, multiple sclerosis, arthritis (for example, rheumatoid arthritis, juvenile arthritis, psoriatic arthritis, arthrosis deformans and Reiter's syndrome), systemic lupus erythematosus (including discoid lupus), psoriasis, ankylosing spondilitis, hepatitis (for example, autoimmunity hepatitis and chronic active hepatitis), inflammatory intestinal disease (for example, ulcerative colitis, Crohn disease and gluten-sensitive intestinal disease), systemic lupus erythematosus, Sjogren's syndrome, Behcet disease, pemphigus, pemphigoid, autoimmune hemolytic anemia, autoimmune inflammatory eye disease, autoimmune neonatal thrombocytopenia, autoimmune neutropenia, autoimmune oophoritis and testitis, autoimmune thrombocytopenia, autoimmune thyroiditis, polymyositis, dermatomyositis, myasthenia gravis, adrenaline agonist resistance, alopecia areata, antiphospholipid syndrome, adrenal autoimmune disease (for example, autoimmune Addison's disease), celiac sprue dermatitis, chronic fatigue immune dysfunction syndrome, (CFIDS), cold agglutinin disease, essential mixed cryoglobulinemia, fibromyalgia-fibromyositis, glomerular nephritis (for example, IgA nephropathy), Grave's disease, hyperthyroidism (such as Hashimoto's thyroiditis), idiopathic thrombocytopenic purpura (ITP), mixed connective tissue disease, Type I or immune-mediated diabetes, pernicious anemia, polychondritis, polyglandular syndrome, stiff-person syndrome, leukoderma, sarcoidosis, polyglandular endocrinopathy, other endocrine gland disorders, atherosclerosis, hepatic fibrosis (such as primary biliary liver cirrhosis), lung fibrosis (such as spontaneous pulmonary fibrosis), chronic obstructive pulmonary disease, dermatosclerosis (including CREST syndrome and Raynaud's phenomenon), endometriosis, uterine adenomyosis, tubulointerstitial nephritis, dense deposit disease, acute kidney injury, myocarditis, cardiomyopathy, neuritis (such as Guillain-Barré syndrome), polyarteritis nodosa, cardiotomy syndrome, chronic inflammatory demyelinating polyneuropathy, IgA neuropathy, lichen planus, Meniere's disease, post-myocardial infarction (post-MI), uveitis, uveitis ophthalmia, vasculitis, primary agammaglobulinemia, cancer (for example, brain tumor, laryngeal cancer, lip/oral cancer, hypopharyngeal cancer, thyroid cancer, esophageal cancer, breast cancer, lung cancer, stomach cancer, adrenocortical carcinoma, cholangiocarcinoma, gallbladder cancer, liver cancer, pancreatic cancer, bladder cancer, colorectal cancer, uterine cancer, ovarian cancer, prostate cancer, testicular cancer, chronic lymphocytic leukemia, chronic myelogenous leukemia, Ewing's tumor, Hodgkin disease, non-Hodgkin lymphoma, melanoma, mesothelioma and multiple myeloma), infection that is resistant immunological elimination (such as severe acute respiratory syndrome (SARS)), lethal cytokine storm accompanying virulent influenza infection, and sepsis, among which asthma, atopic dermatitis, pollen hypersensitivity, anaphylactic shock, dermatosclerosis, Crohn disease, ulcerative colitis, arthritis, systemic lupus erythematosus, ankylosing spondilitis, hepatic fibrosis, pulmonary fibrosis, acute kidney injury, vasculitis and cancer are preferred.

A "stable" pharmaceutical composition according to the invention is one that has no significant observable change at refrigerating temperature (2 to 8°C) for at least 12 months, preferably 2 years and even more preferably 3 years, or at room temperature (22 to 28°C) for at least 3 months, preferably 6 months and even more preferably 1 year. For example, after storage at 5°C for 2 years, no clouding is observed, the OD650 is 0.014 or lower, preferably 0.01 or lower and more preferably 0.008 or lower, the pH drift is 1 or lower, preferably 0.8 or lower and more preferably 0.5 or lower, and the Kd value is -4 mL/g or greater, preferably -2 mL/g or greater and more preferably a positive value, or particles with sizes of 1.5 $\mu$m or greater as measured by a particle counter is no more than 1500 particles/mL, preferably no more than 1000 particles /mL, more preferably no more than 750 particles /mL and most preferably no more than 500 particles /mL..

If necessary, the pharmaceutical composition of the invention may also have a preservative, adsorption inhibitor, soothing agent, sulfur-containing reducing agent or antioxidant added as appropriate.

Preservatives are not particularly restricted so long as they are pharmaceutically acceptable, and examples include methyl paraoxybenzoate, ethyl paraoxybenzoate, sorbic acid, phenol, cresol and chlorocresol.

Adsorption inhibitors are also not particularly restricted so long as they are pharmaceutically acceptable, and examples include human serum albumin, lecithin, dextran, ethylene oxide-propylene oxide copolymer, hydroxypropyl cellulose, methyl cellulose, polyoxyethylene hardened castor oil and polyethylene glycol.

Soothing agents are also not particularly restricted so long as they are pharmaceutically acceptable, and local anesthetics such as lidocaine are examples.

Sulfur-containing reducing agents are also not particularly restricted so long as they are pharmaceutically acceptable, and examples include sulfhydryl group-containing compounds such as N-acetylcysteine, N-acetylhomocysteine, thioctic acid, thiodiglycol, thioethanolamine, thioglycerol, thiosorbitol, thioglycolic acid and its salts, sodium thiosulfate, glutathione, and thioalkanoic acids of 1 to 7 carbon atoms.

Antioxidants are also not particularly restricted so long as they are pharmaceutically acceptable, and examples include erythorbic acid, dibutylhydroxytoluene, butylhydroxyanisole, $\alpha$-tocopherol, tocopherol acetate, L-ascorbic acid and its salts, L-ascorbic acid palmitate, L-ascorbic acid stearate, sodium bisulfite, sodium sulfite, triamyl gallate and propyl gallate, or chelating agents such as disodium ethylenediaminetetraacetate (EDTA), sodium pyrophosphate and sodium metaphosphate.

The pharmaceutical composition of the invention may also be a lyophilized preparation for more prolonged storage.

The invention will now be described in greater detail by the following examples, with the understanding that the scope of the invention is not limited to the examples.

EXAMPLES

Comparative Example 1: Clouding of human anti-IL-33 monoclonal antibody-containing pharmaceutical compositions

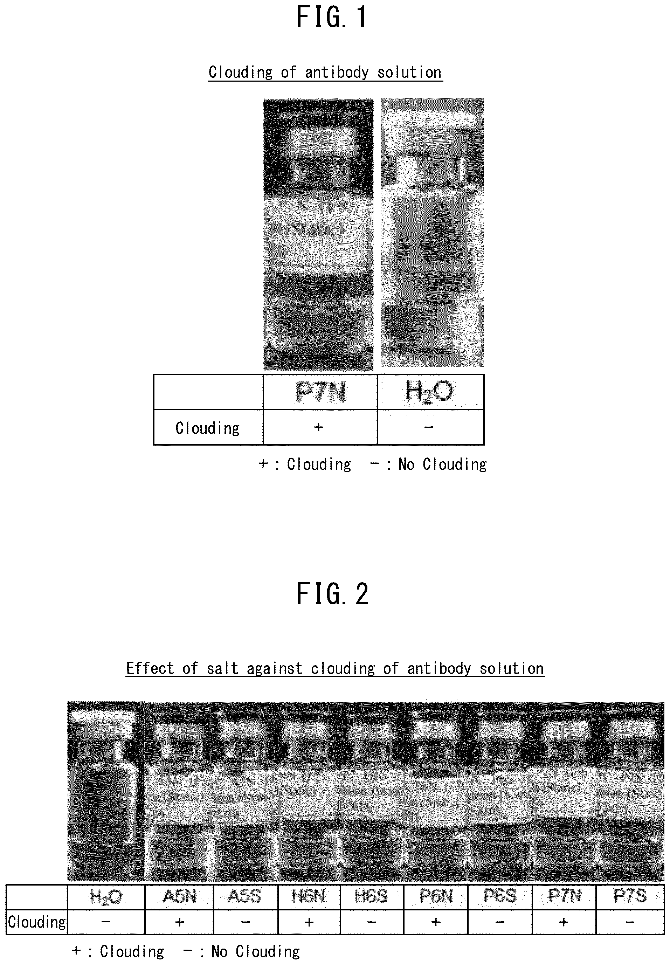

Human anti-IL-33 monoclonal antibody (A10-1C04, A23-1A05, A25-2C02, A25-3H04 or A26-1F02) was prepared to a concentration of 150 mg/mL in a solvent (10 mM Na-phosphate/pH 7/150 mM NaCl/0.02% (w/v) polysorbate 80) (hereunder, "P7N"), and the presence of clouding was confirmed by visual observation (shown in Fig. 1 for A10-1C04). Confirmation of the presence or absence of clouding was by direct observation under white light (13W fluorescent lamp) against a black background. When the pharmaceutical composition was measured for subvisible particles (microparticles of 5 $\mu$m or greater) using a FlowCam (Fluid Imaging Technologies), 1919 particles /mL were detected. These results suggested that each human anti-IL-33 monoclonal antibody (A10-1C04, A23-1A05, A25-2C02, A25-3H04 or A26-1F02) had high aggregation and would be difficult to prepare as a formulation.

Comparative Example 2: Effect of pH on clouding of human anti-IL-33 monoclonal antibody-containing pharmaceutical compositions

In order to improve clouding of each human anti-IL-33 monoclonal antibody (A10-1C04, A23-1A05, A25-2C02, A25-3H04 or A26-1F02), the buffering agent was changed as follows, based on the formulation of Comparative Example 1, to alter the pH of the pharmaceutical composition. The antibody concentration was 150 mg/ml. The following formulations were examined.

- 10 mM Na-acetate/pH 4/150 mM NaCl/0.02% (w/v) polysorbate 80 (hereunder, "A4N")
- 10 mM Na-acetate/pH 5/150 mM NaCl/0.02% (w/v) polysorbate 80 (hereunder, "A5N")
- 10 mM histidine/pH 6/150 mM NaCl/0.02% (w/v) polysorbate 80 (hereunder, "H6N")
- 10 mM Na-phosphate/pH 6/150 mM NaCl/0.02% (w/v) polysorbate 80 (hereunder, "P6N")
- 10 mM Na-phosphate/pH 7/150 mM NaCl/0.02% (w/v) polysorbate 80 (hereunder, "P7N"), and
- 10 mM Na-phosphate/pH 8/150 mM NaCl/0.02% (w/v) polysorbate 80 (hereunder, "P8N")

Evaluation of the properties found clouding in all of the formulations at pH 4, 5, 6, 7 and 8. The results suggested that clouding is not improved even by varying the pH of the formulation.

Evaluation of the properties by direct observation was by the method described for Comparative Example 1.

Example 1: Clouding-inhibiting effect by addition of sugar

In order to improve clouding in each human anti-IL-33 monoclonal antibody (A10-1C04, A23-1A05, A25-2C02, A25-3H04 or A26-1F02), evaluation was conducted with the following formulations, having addition of 5% sorbitol instead of sodium chloride in the formulations of Comparative Example 2 (A4N, A5N, H6N, P6N, P7N and P8N). The antibody concentration was 150 mg/ml.

- 10 mM Na-acetate/pH 4/5% (w/v) sorbitol/0.02% (w/v) polysorbate 80 (hereunder, "A4S")
- 10 mM Na-acetate/pH 5/5% (w/v) sorbitol/0.02% (w/v) polysorbate 80 (hereunder, "A5S")
- 10 mM histidine/pH 6/5% (w/v) sorbitol/0.02% (w/v) polysorbate 80 (hereunder, "H6S")
- 10 mM Na-phosphate/pH 6/5% (w/v) sorbitol/0.02% (w/v) polysorbate 80 (hereunder, "P6S")
- 10 mM Na-phosphate/pH 7/5% (w/v) sorbitol/0.02% (w/v) polysorbate 80 (hereunder, "P7S")
- 10 mM Na-phosphate/pH 8/5% (w/v) sorbitol/0.02% (w/v) polysorbate 80 (hereunder, "P8S")

As a result of evaluating the properties and OD650 (turbidity), clouding was found to be improved by addition of sorbitol instead of sodium chloride (shown in Fig. 2 for A10-1C04). Addition of sorbitol also inhibited increase in OD650 after 3 months at 40°C, with the inhibiting effect being particularly favorable at pH 5 to 7 (Table 3).

Evaluation of the properties by direct observation was by the method described for Comparative Example 1. The OD650 was determined for a 100 μL solution of each formulation, measuring the absorbance at 650 nm using a Molecular Device microplate reader (SoftMax Pro software).

[Table 3]

Table 3: OD650 for each formulation

| Formulation | At preparation | After 3 months storage at 40°C |
|-------------|----------------|--------------------------------|
| A4S | 0.006 | 0.014 |
| A5S | 0.006 | 0.005 |
| H6S | 0.007 | 0.008 |
| P6S | 0.005 | 0.004 |
| P7S | 0.006 | 0.004 |
| P8S | 0.007 | 0.014 |

Example 2: Salt concentration and clouding (1)

After dissolving each human anti-IL-33 monoclonal antibody (A10-1C04, A23-1A05, A25-2C02, A25-3H04 or A26-1F02) in 10 mM histidine/pH 6.0 or pH 5.5 buffering solution and adding 0, 50 or 100 mM NaCl, the interaction parameter (Kd value) was measured as an index of aggregation. The measuring temperature was 25°C, and the antibody concentration was adjusted to 0.5, 1, 2.5, 5, 10 or 20 mg/mL. Since the Kd value was negative (aggregation increased) with NaCl addition of 50 mM or greater, these results suggested that NaCl addition of less than 50 mM is desirable (shown in Table 4 for A10-1C04). The Kd value can be determined by calculating the diffusion coefficient (Dm) by the dynamic light scattering method, determining the slope from a plot of antibody concentration (abscissa) and diffusion coefficient (ordinate), and dividing the slope by the diffusion coefficient (D0) at concentration 0. Specifically, the Kd value is calculated by the following relational expression. A larger positive value for the Kd value corresponds to lower aggregation.

$$Dm = D0(1 + Kd\ Value \times [antibody\ concentration])$$

[Table 4]

Table 4: Salt concentration and interaction parameters (units: mL/g)

| Salt concentration | pH 5.5 | pH 6 |
|--------------------|--------|------|
| 0 mM | 19.2 | 0.17 |
| 50 mM | -4.7 | -6.2 |
| 100 mM | -6.4 | -7.6 |

Example 3: Salt concentration and clouding (2)

After adding NaCl at 0, 5, 10 and 30 mM to 10 mM histidine/pH 6.0/3.6% (w/v) sorbitol, the Kd value was measured as an index of aggregation. The antibody used was 2.5, 5, 10 or 14 mg/mL A10-1C04. Since the Kd value was negative when NaCl was added at 30 mM or greater (the Kd values for NaCl at 0, 5, 10 and 30 mM were 17.3, 7.3, 1.2 and -4.0 mL/g, respectively), this suggested that NaCl addition at less than 30 mM is desirable. The Kd value was calculated by the method described in Example 2.

Example 4: pH drift

The pH was measured during prolonged storage of pharmaceutical compositions containing each human anti-IL-33 monoclonal antibody (A10-1C04, A23-1A05, A25-2C02, A25-3H04 or A26-1F02). The antibody concentration was 150 mg/mL, and formulations with pH 4 to 8 were examined (A4S, A5S, H6S, P6S, P7S, P8S). The pH of each formulation was measured at the time of preparation and after storage at 40°C for 2, 4, 8 and 12 weeks. As a result, the drift in pH was notable with the formulations at pH 4 (A4S) and pH 8 (P8S) (shown in Table 5 for A10-1C04). It was therefore concluded that the formulations at pH 5 to 7 which had low pH drift were favorable.

[Table 5]

Table 5: pH drift during storage at 40°C

| Formulation | At preparation | 2 weeks | 4 weeks | 8 weeks | 12 weeks | pH drift |
|---|---|---|---|---|---|---|
| A4S | 4.0 | 4.6 | 5.0 | 5.0 | 5.1 | +1.1 |
| A5S | 5.0 | 5.3 | 5.5 | 5.5 | 5.6 | +0.6 |
| H6S | 6.0 | 6.2 | 6.3 | 6.3 | 6.4 | +0.4 |
| P6S | 6.1 | 6.2 | 6.2 | 6.3 | 6.3 | +0.2 |
| P7S | 7.0 | 6.8 | 6.8 | 6.7 | 6.8 | -0.2 |
| P8S | 8.1 | 7.6 | 7.2 | 7.2 | 7.3 | -0.8 |

Example 5: Antibody concentration and viscosity

Pharmaceutical compositions were prepared with each human anti-IL-33 monoclonal antibody (A10-1C04, A23-1A05, A25-2C02, A25-3H04 or A26-1F02) at antibody concentrations of 15, 50, 100, 125, 150, 175 and 200 mg/ml, formulated with 10 mM histidine/pH 6/3.6% (w/v) sorbitol/0.02% (w/v) polysorbate 80. The viscosity was measured at a measuring temperature of 25°C using a viscometer (Model DV3TLVCJ0 Viscometer by Brookfield (CPA-40Z spindle, CPA-44YZ sample cup)). The viscosities of A10-1C04 at antibody concentrations of 15, 50, 100, 125, 150, 175 and 200 mg/mL were 1.19, 1.84, 5.33, 9.15, 16.29, 47.98 and 84.96 cP, respectively, showing that the viscosity increased drastically when 150 mg/mL was exceeded. When solutions with each viscosity were prepared and the injectability with a glass syringe was examined, administration was considered to be somewhat difficult when the viscosity exceeded approximately 20 cP, suggesting that an antibody concentration of less than 175 mg/ml is favorable.

Example 6: Sugar addition and aggregate evaluation, and inhibiting effect on microparticle generation

A composition of each human anti-IL-33 monoclonal antibody (A10-1C04, A23-1A05, A25-2C02, A25-3H04 or A26-1F02) with 10 mM histidine/pH 6.0 was prepared to antibody concentrations of 0.6125, 1.25, 2.5, 5 and 10 mg/mL, and the effect of sucrose or sorbitol addition on the Kd value as an index of aggregation was confirmed. The Kd value was calculated by the method described in Example 2. For example, the Kd value when using A10-1C04 was 33.4 mL/g without sugar addition, while addition of 3.6% (w/v) sucrose or sorbitol resulted in positive values of 24.7 and 33.5 mL/g, respectively. These results suggested that addition of sucrose is instead of sorbitol is also favorable. A formulation with addition of 3.6% (w/v) sorbitol, with an antibody concentration of 10 mg/ml (10 mM histidine/pH 6.0/3.6% (w/v) sorbitol), was measured for microparticles of sizes 1.5 $\mu$m or greater at the time of preparation and upon storage for 1 week at 50°C, using a particle counter HIAC (Model System 9703+ by HACH). Measurement with the HIAC was carried out 4 times for each specimen at an injection volume of 100 $\mu$L, rejecting the first data. As a result, addition of sorbitol at 3.6% (w/v) inhibited increase in the number of microparticles compared to no addition of sorbitol, thus suggesting that an aggregation inhibiting effect is exhibited by addition of sorbitol (shown in Table 6 for A10-1C04).

[Table 6]

Table 6: Inhibiting effect on microparticle generation by sorbitol (units: num/100 µL)

| Saccharide | Particle size (µm) | At preparation | After 1 week storage at 50°C |
|---|---|---|---|
| Not added | 1.5 to 2.0 | 10.9 | 98.1 |
| | 2.0 to 5.0 | 8.2 | 67.1 |
| | 5.0 to 10 | 1.2 | 7.4 |
| | 10 to 25 | 0.3 | 2.9 |
| | 25 to | 0.1 | 0.0 |
| 3.6% (w/v) sorbitol | 1.5 to 2.0 | 31.1 | 22.8 |
| | 2.0 to 5.0 | 13.0 | 18.0 |
| | 5.0 to 10 | 3.2 | 2.9 |
| | 10 to 25 | 1.2 | 0.6 |
| | 25 to | 0.0 | 0.0 |

Example 7: Aggregation inhibiting effect of surfactant

A composition of 10 mg/mL of each human anti-IL-33 monoclonal antibody (A10-1C04, A23-1A05, A25-2C02, A25-3H04 or A26-1F02) with 10 mM histidine/pH 6/3.6% (w/v) sorbitol was prepared, and the effect on aggregation by addition of polysorbate 20 or polysorbate 80 as a surfactant at 0.02% (w/v) was confirmed. No aggregation was observed at the time of preparation, regardless of whether a surfactant was added.

Example 8: Stability of subcutaneous formulation and intravenous formulation

An intravenous formulation (10 mg/mL antibody/10 mM histidine/pH 6/3.6% (w/v) sorbitol/0.02% (w/v) polysorbate 80) and a subcutaneous formulation (150 mg/mL antibody/10 mM histidine/pH 6/4% (w/v) sorbitol/0.02% (w/v) polysorbate 80) was prepared for each human anti-IL-33 monoclonal antibody (A10-1C04, A23-1A05, A25-2C02, A25-3H04 or A26-1F02). When the intravenous formulations were examined, no clouding, aggregated microparticle number increase or pH drift were found even after storage for 24 months at 5°C, thus confirming that they were stable. Similar stability can be evaluated for the subcutaneous formulations as well.

Example 9: Effect of pH on clouding of human anti-IL-33 monoclonal antibody-containing pharmaceutical compositions (2)

In order to improve clouding in each human anti-IL-33 monoclonal antibody (A10-1C04, A23-1A05, A25-2C02, A25-3H04 or A26-1F02), the following formulations were prepared without sodium chloride, within the pH ranges of the formulations confirmed in Comparative Example 2.

- 10 mM Na-acetate/pH 4/0.02% (w/v) polysorbate 80 (hereunder, "A4")
- 10 mM Na-acetate/pH 5/0.02% (w/v) polysorbate 80 (hereunder, "A5")
- 10 mM histidine/pH 6/0.02% (w/v) polysorbate 80 (hereunder, "H6")
- 10 mM histidine/pH 7/0.02% (w/v) polysorbate 80 (hereunder, "H7")
- 10 mM Na-phosphate/pH 8/0.02% (w/v) polysorbate 80 (hereunder, "P8")

When the properties of 150 mg/mL A10-1C04 were evaluated, for example, it was found that clouding was improved by not adding sodium chloride. The inhibiting effect was particularly favorable at pH 4 to 7 (Table 7).

[Table 7]

Table 7: Formulation property

| Formulation | Property |
|---|---|
| A4 | - |
| A5 | - |
| H6 | - |
| H7 | - |
| P8 | + |
| -: Clear (no clouding) + : clouding | |

Example 10: Effect of salt on clouding of human anti-IL-33 monoclonal antibody-containing pharmaceutical compositions (2)

In order to improve clouding in each human anti-IL-33 monoclonal antibody (A10-1C04, A23-1A05, A25-2C02, A25-3H04 or A26-1F02), evaluation was conducted using the formulations of Example 9 (A4, A5 and H6), with addition of sodium chloride at different concentrations.

- 10 mM Na-acetate/pH 4/10 mM NaCl/0.02% (w/v) polysorbate 80 (hereunder, "A4N10")
- 10 mM Na-acetate/pH 4/30 mM NaCl/0.02% (w/v) polysorbate 80 (hereunder, "A4N30")
- 10 mM Na-acetate/pH 4/50 mM NaCl/0.02% (w/v) polysorbate 80 (hereunder, "A4N50")
- 10 mM Na-acetate/pH 4/100 mM NaCl/0.02% (w/v) polysorbate 80 (hereunder, "A4N100")
- 10 mM Na-acetate/pH 5/10 mM NaCl/0.02% (w/v) polysorbate 80 (hereunder, "A5N10")
- 10 mM Na-acetate/pH 5/30 mM NaCl/0.02% (w/v) polysorbate 80 (hereunder, "A5N30")
- 10 mM Na-acetate/pH 5/50 mM NaCl/0.02% (w/v) polysorbate 80 (hereunder, "A5N50")
- 10 mM Na-acetate/pH 5/100 mM NaCl/0.02% (w/v) polysorbate 80 (hereunder, "A5N100")
- 10 mM histidine/pH 6/10 mM NaCl/0.02% (w/v) polysorbate 80 (hereunder, "H6N10")
- 10 mM histidine/pH 6/30 mM NaCl/0.02% (w/v) polysorbate 80 (hereunder, "H6N30")
- 10 mM histidine/pH 6/50 mM NaCl/0.02% (w/v) polysorbate 80 (hereunder, "H6N50")
- 10 mM histidine/pH 6/100 mM NaCl/0.02% (w/v) polysorbate 80 (hereunder, "H6N100") When the properties of 150 mg/mL A10-1C04 were evaluated, for example, it was found that clouding was inhibited with 10 mM sodium chloride at pH 4 to 6, whereas clouding was observed when sodium chloride exceeded 30 mM (Table 8).

[Table 8]

Table 8: Formulation property

| | Without | N10 | N30 | N50 | N100 |
|---|---|---|---|---|---|
| A4 | - | - | + | + | + |
| A5 | - | - | + | + | + |
| H6 | - | - | + | + | + |
| -: Clear (no clouding) + : clouding | | | | | |

Example 11: Aggregation inhibiting effect of surfactant (2)

In order to confirm the effect of a surfactant on 10 mg/mL of each human anti-IL-33 monoclonal antibody (A10-1C04, A23-1A05, A25-2C02, A25-3H04 or A26-1F02), the following formulations were prepared with addition of a surfactant. Each sample was then rotated at 30 rpm using a rotator in a thermostatic chamber at 50°C. The sample was periodically removed and visually examined for 5 seconds each under a 2000 to 3750 Lux fluorescent lamp against white and black board backgrounds. Numerous aggregates were observed after 2 days in the samples without surfactant addition, but no aggregates were observed even after 7 days in the samples with addition of polysorbate 80 and poloxamer 188, and in particular, no aggregates were observed even after 14 days in the samples with addition of polysorbate 80 at 0.02% or greater. The samples with addition of polysorbate 20 had no visible particles up to 4 days later, and while exhibiting a small amount of visible particles after 7 days, the visible particles were no longer observed after 14 days. The results for A10-1C04 are shown in Table 9.

    10 mM histidine/pH 6 (hereunder, "H6(-)")
    10 mM histidine/pH 6/0.02% (w/v) poloxamer 188 (hereunder, "H6PX")
    10 mM histidine/pH 6/0.02% (w/v) polysorbate 20 (hereunder, "H6P20")
    10 mM histidine/pH 6/0.01% (w/v) polysorbate 80 (hereunder, "H6PS1")
    10 mM histidine/pH 6/0.02% (w/v) polysorbate 80 (hereunder, "H6PS2")
    10 mM histidine/pH 6/0.05% (w/v) polysorbate 80 (hereunder, "H6PS5")


[Table 9]

Table 9: Aggregation observed in specimens stored at 50°C, 30 rpm

|  | Day 0 | Day 1 | Day 2 | Day 3 | Day 7 | Day 14 |
|---|---|---|---|---|---|---|
| H6(-) | - | - | ++ | ++ | ++ | ++ |
| H6PX | - | - | - | - | - | + |
| H6P20 | - | - | - | -* | + | - |
| H6PS1 | - | - | - | - | - | + |
| H6PS2 | - | - | - | - | - | - |
| H6PS5 | - | - | - | - | - | - |
| - : No aggregation<br>+ : Slight aggregation confirmed<br>++: Significant aggregation confirmed<br>*: Removed after 4 days | | | | | | |

Example 12: Evaluation of sugar addition and aggregation (2)

Compositions of 10 mg/mL of each human anti-IL-33 monoclonal antibody (A10-1C04, A23-1A05, A25-2C02, A25-3H04 or A26-1F02) were prepared with 10 mM histidine/pH 6.0, and drug solutions were prepared with addition of mannitol, trehalose, sucrose or sorbitol. Each sample was then stored frozen at -80°C for at least 8 hours, and then allowed to stand for at least 4 hours at room temperature to thaw. This procedure was repeated 6 times. It was then visually examined for 5 seconds each under a 2000 to 3750 Lux fluorescent lamp against white and black board backgrounds. Addition of a sugar lowered the amount of aggregates after freezing and thawing compared to no addition, especially inhibiting effect on aggregation was observed when sorbitol and sucrose were used. The results for A10-1C04 are shown in Table 10.

    10 mM histidine/pH 6 (hereunder, "H6(-)")
    10 mM histidine/pH 6/3.0% (w/v) sorbitol (hereunder, "H6So3")
    10 mM histidine/pH 6/3.6% (w/v) sorbitol (hereunder, "H6So3.6")
    10 mM histidine/pH 6/4.0% (w/v) sorbitol (hereunder, "H6So4")
    10 mM histidine/pH 6/5.0% (w/v) sorbitol (hereunder, "H6So5")
    10 mM histidine/pH 6/3.6% (w/v) sucrose (hereunder, "H6Su")
    10 mM histidine/pH 6/3.6% (w/v) trehalose (hereunder, "H6Tr")
    10 mM histidine/pH 6/3.6% (w/v) mannitol (hereunder, "H6Ma")

[Table 10]

Table 10: Aggregation observed by visual inspection after freezing and thawing

|  | Freeze/thaw (cycle) | |
|---|---|---|
|  | 0 | 6 |
| H6(-) | - | ++ |
| H6So3 | - | - |
| H6So3.6 | - | - |
| H6So4 | - | - |
| H6So5 | - | - |
| H6Su | - | - |
| H6Tr | - | + |
| H6 Ma | - | + |
| - : No aggregation<br>+ : Slight aggregation confirmed<br>++: Significant aggregation confirmed | | |

Example 13: Lyophilization

Compositions of 150 mg/mL of each human anti-IL-33 monoclonal antibody (A10-1C04, A23-1A05, A25-2C02, A25-3H04 or A26-1F02) were prepared with 10 mM histidine/pH 6.00.02% (w/v) polysorbate 80, and after addition of mannitol, trehalose, sucrose or sorbitol, the compositions were lyophilized with a shelf-type freeze drier (product of Kyowa Vacuum Engineering). The caked form after lyophilization was visually confirmed.

Reconstitution was determined by confirming that no caking remained after adding water for injection and allowing the mixture to stand for half a day at 5°C. All of the samples had satisfactory cake shape, with redissolution confirmed after addition of water for injection. This confirmed that it is possible to obtain lyophilized preparations. The results for A10-1C04 are shown in Table 11.

10 mM histidine/pH 6/0.02% (w/v) polysorbate 80/4.0% (w/v) sorbitol (hereunder, "LYSO")
10 mM histidine/pH 6/0.02% (w/v) polysorbate 80/4.0% (w/v) sucrose (hereunder, "LYSU")
10 mM histidine/pH 6/0.02% (w/v) polysorbate 80/4.0% (w/v) trehalose (hereunder, "LYTR")
10 mM histidine/pH 6/0.02% (w/v) polysorbate 80/2.0% (w/v) mannitol (hereunder, "LYMA")

[Table 11]

Table 11: Physical properties after lyophilization

|  | LYSO | LYSU | LYTR | LYMA |
|---|---|---|---|---|
| Outer appearance | Good | Good | Good | Good |
| Resolubility | Sol | Sol | Sol | Sol |
| Good: caking formed, Bad: no caking formed, Sol: dissolved,<br>Dis: not dissolved | | | | |

Example 14: Stability test (1)

Compositions of 10 mg/mL of each human anti-IL-33 monoclonal antibody (A10-1C04, A23-1A05, A25-2C02, A25-3H04 or A26-1F02) were prepared with 10 mM histidine/pH 6/0.02% (w/v) polysorbate 80/3.6% (w/v) sorbitol, and then each was filled into a glass vial and sealed with a halogenated butyl rubber stopper, and stored for 1, 2 and 3 years at a temperature of 2 to 8°C as a long-term stability test. The presence or absence of aggregation was confirmed by visual examination for 5 seconds each under a 2000 to 3750 Lux fluorescent lamp, against white and black board backgrounds. The binding activity was evaluated by the following method. Human IL-33 was added to a 96-well plate and allowed to form a solid phase overnight. BSA was used for blocking, and then the sample solution was added to each well, reacted with HRP-labeled anti-human IgG antibody and colored with TMB. The absorbance at 450 nm and 650 nm was then measured with a plate reader (Molecular Devices Corp.) to determine the EC50 value. The EC50 value of the standard solution was also determined in the same manner, and the ratio was calculated. No aggregation or pH drift was observed at any of the measurement points, and antibody binding activity was not reduced. The results for A10-1C04 are shown in Table 12.

[Table 12]

Table 12: Long-term stability test (1)

|  | 2 to 8°C T0 | 2 to 8°C, 1 year | 2 to 8°C, 2 years | 2 to 8°C, 3 years |
|---|---|---|---|---|
| Visual inspection | - | - | - | - |
| Binding activity | 100% | 110% | 101% | 101% |
| pH | 6.0 | 6.0 | 6.0 | 6.0 |
| -: No aggregation<br>+: Slight aggregation confirmed<br>++: Significant aggregation confirmed | | | | |

Example 15: Stability test (2)

Compositions of 150 mg/mL of each human anti-IL-33 monoclonal antibody (A10-1C04, A23-1A05, A25-2C02, A25-3H04 or A26-1F02) were prepared with 10 mM histidine/pH 6/0.02% (w/v) polysorbate 80/4.0% (w/v) sorbitol, and then each was filled into a glass vial and sealed with a halogenated butyl rubber stopper, and stored for 3 and 6 months at a temperature of 2 to 8°C as a long-term stability test. The evaluation was conducted by the same method as Example 14. No aggregation or pH drift was observed at any of the measurement points, and antibody binding activity was not reduced. The results for A10-1C04 are shown in Table 13.

[Table 13]

Table 13: Long-term stability test (2)

|  | 2 to 8°C T0 | 2 to 8°C, 3 months | 2 to 8°C, 6 months |
|---|---|---|---|
| Visual inspection | - | - | - |
| Binding activity | 115% | 110% | 100% |
| pH | 5.9 | 5.8 | 5.8 |
| -: No aggregation<br>+: Slight aggregation confirmed<br>++: Significant aggregation confirmed | | | |

**Claims**

1. A pharmaceutical composition comprising human anti-IL-33 monoclonal antibody as an active ingredient, wherein: the combination of amino acid sequences in the heavy chain complementarity determining region 1 (H1), the heavy chain complementarity determining region 2 (H2), the heavy chain complementarity determining region 3 (H3), the light chain complementarity determining region 1 (L1), the light chain complementarity determining region 2 (L2) and the light chain complementarity determining region 3 (L3) of the human anti-IL-33 monoclonal antibody is one from among C1 to C5 listed in Table 1, and the pharmaceutical composition contains substantially no sodium chloride or contains sodium chloride at less than 30 mM.

[Table 1]

|    | H1 | H2 | H3 | L1 | L2 | L3 |
|----|----|----|----|----|----|----|
| C1 | SEQ ID NO: 11 | SEQ ID NO: 12 | SEQ ID NO: 13 | SEQ ID NO: 14 | SEQ ID NO: 15 | SEQ ID NO: 16 |
| C2 | SEQ ID NO: 17 | SEQ ID NO: 18 | SEQ ID NO: 19 | SEQ ID NO: 20 | SEQ ID NO: 21 | SEQ ID NO: 22 |
| C3 | SEQ ID NO: 17 | SEQ ID NO: 23 | SEQ ID NO: 24 | SEQ ID NO: 25 | SEQ ID NO: 26 | SEQ ID NO: 27 |
| C4 | SEQ ID NO: 28 | SEQ ID NO: 29 | SEQ ID NO: 30 | SEQ ID NO: 25 | SEQ ID NO: 31 | SEQ ID NO: 32 |
| C5 | SEQ ID NO: 17 | SEQ ID NO: 33 | SEQ ID NO: 34 | SEQ ID NO: 35 | SEQ ID NO: 36 | SEQ ID NO: 37 |

2. The pharmaceutical composition according to claim 1, wherein the combination of amino acid sequences of the human anti-IL-33 monoclonal antibody heavy chain variable region and light chain variable region is any one from among V1 to V5 listed in Table 2.

[Table 2]

|    | Heavy chain variable region | Light chain variable region |
|----|----|----|
| V1 | SEQ ID NO: 38 | SEQ ID NO: 39 |
| V2 | SEQ ID NO: 40 | SEQ ID NO: 41 |
| V3 | SEQ ID NO: 42 | SEQ ID NO: 43 |
| V4 | SEQ ID NO: 44 | SEQ ID NO: 45 |
| V5 | SEQ ID NO: 46 | SEQ ID NO: 47 |

3. The pharmaceutical composition according to claim 1 or 2, wherein the human anti-IL-33 monoclonal antibody is A10-1C04, A23-1A05, A25-2C02, A25-3H04 or A26-1F02.

4. The pharmaceutical composition according to any one of claims 1 to 3, wherein the sodium chloride concentration is 10 mM or lower.

5. The pharmaceutical composition according to any one of claims 1 to 4, which contains substantially no sodium chloride.

6. The pharmaceutical composition according to any one of claims 1 to 5, wherein the pH is adjusted to be higher than 4 and lower than 8.

7. The pharmaceutical composition according to any one of claims 1 to 6, wherein the pH is adjusted to be 5 to 7.

8. The pharmaceutical composition according to any one of claims 1 to 7, wherein the pH is adjusted by an acetate, histidine or phosphate buffer.

9. The pharmaceutical composition according to any one of claims 1 to 8, wherein the pH is adjusted by histidine.

10. The pharmaceutical composition according to any one of claims 1 to 9, wherein the concentration of the active ingredient is less than 175 mg/ml.

11. The pharmaceutical composition according to any one of claims 1 to 10, wherein the concentration of the active ingredient is 150 mg/ml or lower.

12. The pharmaceutical composition according to any one of claims 1 to 11, which contains at least one polyol.

13. The pharmaceutical composition according to claim 12, wherein the polyol is a saccharide selected from the group consisting of disaccharides and sugar alcohols.

14. The pharmaceutical composition according to claim 12 or 13, wherein the polyol is 3 to 5% (w/v) sorbitol.

15. The pharmaceutical composition according to any one of claims 1 to 14, which contains a surfactant.

16. The pharmaceutical composition according to claim 15, wherein the surfactant is a nonionic surfactant.

17. The pharmaceutical composition according to claim 16, wherein the surfactant is polysorbate 20, polysorbate 80 or poloxamer 188.

18. The pharmaceutical composition according to any one of claims 1 to 17, which includes 10 mM histidine, 4% (w/v) sorbitol, 0.02% (w/v) polysorbate 80 and 150 mg/ml of the active ingredient, and has the pH adjusted to 5.5 to 6.5.

19. The pharmaceutical composition according to claim 18, which is for subcutaneous administration.

20. The pharmaceutical composition according to any one of claims 1 to 17, which includes 10 mM histidine, 3.6% (w/v) sorbitol, 0.02% (w/v) polysorbate 80 and 10 mg/ml of an active ingredient, and has the pH adjusted to 5.5 to 6.5.

21. The pharmaceutical composition according to claim 20, which is for intravenous administration.

22. The pharmaceutical composition according to any one of claims 1 to 21, wherein the active ingredient is A10-1C04.

23. A lyophilized preparation of a pharmaceutical composition according to any one of claims 1 to 22.

24. A pharmaceutical composition comprising human anti-IL-33 monoclonal antibody as an active ingredient, wherein: the combination of amino acid sequences in the heavy chain complementarity determining region 1 (H1), the heavy chain complementarity determining region 2 (H2), the heavy chain complementarity determining region 3 (H3), the light chain complementarity determining region 1 (L1), the light chain complementarity determining region 2 (L2) and the light chain complementarity determining region 3 (L3) of the human anti-IL-33 monoclonal antibody is one from among C1 to C5 listed in Table 1, and the pharmaceutical composition contains a buffering agent, a nonionic surfactant and a polyol.

[Table 3]

|  | H1 | H2 | H3 | L1 | L2 | L3 |
|---|---|---|---|---|---|---|
| C1 | SEQ ID NO: 11 | SEQ ID NO: 12 | SEQ ID NO: 13 | SEQ ID NO: 14 | SEQ ID NO: 15 | SEQ ID NO: 16 |
| C2 | SEQ ID NO: 17 | SEQ ID NO: 18 | SEQ ID NO: 19 | SEQ ID NO: 20 | SEQ ID NO: 21 | SEQ ID NO: 22 |
| C3 | SEQ ID NO: 17 | SEQ ID NO: 23 | SEQ ID NO: 24 | SEQ ID NO: 25 | SEQ ID NO: 26 | SEQ ID NO: 27 |
| C4 | SEQ ID NO: 28 | SEQ ID NO: 29 | SEQ ID NO: 30 | SEQ ID NO: 25 | SEQ ID NO: 31 | SEQ ID NO: 32 |
| C5 | SEQ ID NO: 17 | SEQ ID NO: 33 | SEQ ID NO: 34 | SEQ ID NO: 35 | SEQ ID NO: 36 | SEQ ID NO: 37 |

# FIG. 1

Clouding of antibody solution

| | P7N | $H_2O$ |
|---|---|---|
| Clouding | + | − |

+ : Clouding  − : No Clouding

# FIG. 2

Effect of salt against clouding of antibody solution

| | $H_2O$ | A5N | A5S | H6N | H6S | P6N | P6S | P7N | P7S |
|---|---|---|---|---|---|---|---|---|---|
| Clouding | − | + | − | + | − | + | − | + | − |

+ : Clouding  − : No Clouding

**EP 3 851 121 A1**

<table>
<tr><td colspan="2" style="text-align:center"><strong>INTERNATIONAL SEARCH REPORT</strong></td><td colspan="2">International application No.<br>PCT/JP2019/036239</td></tr>
</table>

A. CLASSIFICATION OF SUBJECT MATTER

Int.Cl. A61K39/395(2006.01)i, A61K9/08(2006.01)i, A61K9/19(2006.01)i, A61K47/02(2006.01)i, A61K47/10(2006.01)i, A61K47/12(2006.01)i, A61K47/18(2006.01)i, A61K47/26(2006.01)i, A61P11/06(2006.01)i, A61P15/00(2006.01)i, A61P17/00(2006.01)i, A61P37/08(2006.01)i

According to International Patent Classification (IPC) or to both national classification and IPC

B. FIELDS SEARCHED

Minimum documentation searched (classification system followed by classification symbols)

Int.Cl. A61K39/395, A61K9/08, A61K9/19, A61K47/02, A61K47/10, A61K47/12, A61K47/18, A61K47/26, A61P11/06, A61P15/00, A61P17/00, A61P37/08

Documentation searched other than minimum documentation to the extent that such documents are included in the fields searched

| | |
|---|---|
| Published examined utility model applications of Japan | 1922–1996 |
| Published unexamined utility model applications of Japan | 1971–2019 |
| Registered utility model specifications of Japan | 1996–2019 |
| Published registered utility model applications of Japan | 1994–2019 |

Electronic data base consulted during the international search (name of data base and, where practicable, search terms used)

CAplus/MEDLINE/EMBASE/BIOSIS (STN), 医中誌 WEB (Ichushi WEB)

C. DOCUMENTS CONSIDERED TO BE RELEVANT

| Category* | Citation of document, with indication, where appropriate, of the relevant passages | Relevant to claim No. |
|---|---|---|
| Y | JP 2017-8003 A (MITSUBISHI TANABE PHARMA CORPORATION) 12 January 2017, example 21, table 13 (Family: none) | 1-24 |
| Y | WO 2015/099175 A1 (MITSUBISHI TANABE PHARMA CORPORATION) 02 July 2015, example 21, table 13 & US 2016/0289322 A1, example 21, table 13 & EP 3088517 A1 & KR 10-2016-0101940 A & CN 105980556 A | 1-24 |

☒ Further documents are listed in the continuation of Box C.　☐ See patent family annex.

| | |
|---|---|
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search<br>04 December 2019 (04.12.2019) | Date of mailing of the international search report<br>17 December 2019 (17.12.2019) |
|---|---|
| Name and mailing address of the ISA/<br>　Japan Patent Office<br>　3-4-3, Kasumigaseki, Chiyoda-ku,<br>　Tokyo 100-8915, Japan | Authorized officer<br><br>Telephone No. |

Form PCT/ISA/210 (second sheet) (January 2015)

57

**EP 3 851 121 A1**

**INTERNATIONAL SEARCH REPORT**

International application No.

PCT/JP2019/036239

C (Continuation).   DOCUMENTS CONSIDERED TO BE RELEVANT

| Category* | Citation of document, with indication, where appropriate, of the relevant passages | Relevant to claim No. |
|---|---|---|
| Y | 内山 進，バイオ医薬品の分析のコツ 品質評価のための基礎と応用 第6回タンパク質溶液の性質, PHARM TECH JAPAN, January 2018, vol. 34, no. 1, pp. 109-120, ISSN 0910-4739, in particular, pp. 117-118, "(1) Relationship between composition and stability of protein solution", non-official translation (UCHIYAMA, Susumu, "Tips for analyzing biopharmaceuticals, and fundamentals and applications for quality evaluation, The 6th: Properties of protein solution") | 1-24 |
| Y | 本田 真也 ほか，バイオ医薬品の分析のコツ 品質評価のための基礎と応用 第9回タンパク質の安定性分析と安定化設計（後編）, PHARM TECH JAPAN, April 2018, vol. 34, no. 5, pp. 885-894, ISSN 0910-4739, in particular, pp. 887-889, "(1) Trends over ages", fig. 3-8, non-official translation (HONDA, Shinya et al., "Tips for analyzing biopharmaceuticals, and fundamentals and applications for quality evaluation, The 9th: stability analysis and stabilization design of protein (Second part)") | 1-24 |
| Y | 伊豆津 健一，タンパク質医薬品の凍結乾燥, 薬剤学, 2012, vol. 72, no. 6, pp. 353-358, ISSN 0372-7629, in particular, pp. 354-356, "3. Stability changing factor and formulation design", table 3, (IZUTSU, Kenichi, "An Introduction to Freeze-Drying of Protein Pharmaceuticals", Journal of Pharmaceutical Science and Technology, Japan) | 1-24 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2015099175 A

- US 4816567 A

**Non-patent literature cited in the description**

- **KOHLER et al.** *Nature,* 1975, vol. 256, 495
- **CLACKSON et al.** *Nature,* 1991, vol. 352, 624-628
- **MARKS et al.** *J. Mol. Biol.,* 1991, vol. 222, 581-597
- **KABAT et al.** Sequences of Proteins of Immunological Interest. Public Health Service, National Institutes of Health, 1991

- Peptide and Protein Drug Delivery. Marcel Dekker, Inc, 1991, 247-301
- **JONES, A.** *Adv. Drug Delivery Rev.,* 1993, vol. 10, 29-90
- **REY ; MAY.** *Freeze-Drying/Lyophilization of Pharmaceutical & Biological Products,* 2004, ISBN 0824748689